# EUROPEAN PATENT SPECIFICATION

(11) **EP 3 962 368 B1**
(45) Date of publication and mention of the grant of the patent: **07.06.2023**
(21) Application number: 20742445.8
(22) Date of filing: 04.05.2020
(51) Int. Cl.: A61B 17/00

(54) **CROSSABLE INTERSEPTAL OCCLUDER DEVICE**
DURCHQUERBARE INTERSEPTALE VERSCHLUSSVORRICHTUNG
DISPOSITIF D'OCCLUSION INTERSEPTALE POUVANT ÊTRE TRAVERSÉ

(30) Priority: 03.05.2019 IT 201900006534
(43) Date of publication of application: 09.03.2022
(73) Proprietor: RECROSS CARDIO, INC., Kent County, DE 19901 (US)
(72) Inventor: ROLANDO, Giovanni, Dover Kent County, DE 19901 (US); ANTONUCCI, Marta, Dover Kent County, DE 19901 (US); ROGGERO, Simona, Dover Kent County, DE 19901 (US)
(74) Representative: Crippa, Paolo Ernesto
(86) International application number: PCT/IB2020/054195
(87) International publication number: WO 2020/225698

(56) References cited:
- US-A1- 2007 073 337
- US-A1- 2014 012 368
- US-A1- 2016 022 423
- US-A1- 2016 296 684

## Description

### . Field of the invention

In the most general aspect thereof, the present invention is directed to an occluder device. In particular, the present invention relates to an interseptal occluder device. Even more particularly, the present invention relates to an interseptal occluder device of the crossable type, or crossable interseptal occluder device.

In particular, the present invention is directed to a device for closing a defect of a partition or an opening obtained in a partition, not necessarily of the heart.

Even more particularly, the present invention is directed to a device for closing a defect present in a partition, for example a defect in an atrial partition, so that the same defect, even if occluded, can then be used for a medical device to pass through said defect.

. Moreover, the device of the present invention is intended to occlude defects (typically congenital defects, but not only) or interatrial partition holes/openings created following percutaneous interventions with trans-septal puncture techniques (for example for the mitral valve repair or the occlusion of the Left Atrial Appendage).

### . Background art

A partition is for example a thin wall dividing a cavity into two smaller cavities or chambers or compartments. The term "partition" is intended to define both a heart wall which divides two atria, as well as a wall which divides the right or left atrium and ventricle.

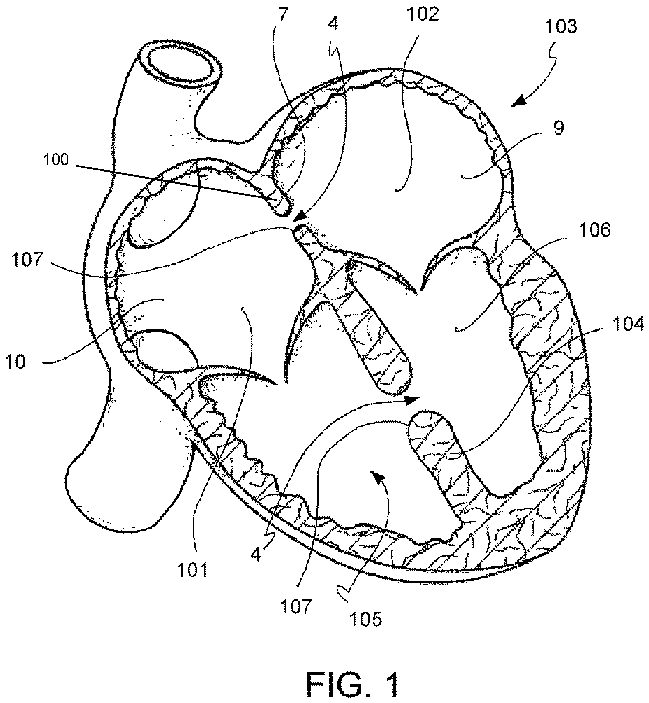

With reference to Figure 1, an atrial partition 100 is a tissue wall which separates the right atrium 101 from the left atrium 102 of the heart 103.

A ventricular partition 104 is a tissue wall which separates the right 105 and left 106 ventricles of the heart 103.

A defect 107 of the partition 100, 104 may include a perforation or a hole in the partition. A defect 107 of the partition 100, 104 can occur congenitally or by piercing the partition with a medical device to access a position within the heart.

The femoral vein is an access point for many laboratory catheterization procedures, with a smaller percentage of procedures using the access to arteries.

The atrial partition 100 is a percutaneous access point, for example for atrial fibrillation therapy, closure of the left atrial appendage, percutaneous repair of the mitral valve, and percutaneous replacement of the mitral valve. In these and other procedures, the devices need to cross the atrial partition 100 and, in doing so, can leave an orifice in the atrial partition which cannot close or heal on its own.

Therefore, these defects are often closed using devices, such as clips or occluders. However, these devices do not allow re-crossing through the partition.

Therefore, there is a need for improved occlusion devices for closing a defect or an opening of the partition, and for re-crossing it in (possible) subsequent procedures.

Crossable occluding devices are known from the prior art. These have an anchoring structure and a diaphragm or valve connected thereto.

For example, documents WO2017136287A1**,** US2014074155 show crossable occluder solutions.

Document US20070073337 shows devices and methods for occluding the defects of internal tissues, such us the defects of the partition, with clip-based devices. A device having a clip structure is shown, which includes a tubular body having at least a first and a second deflectable element coupled thereto. The first and second elements are coupled at the opposite ends of the tubular body and configured to switch from a non-deployed configuration to a deployed configuration. In the deployed configuration, each element extends outwardly away from the tubular body in a position configured to rest on a tissue surface. The first and second elements of the clip are preferably configured to support a tissue wall therebetween and close any opening in the tissue wall. This solution does not allow to re-cross the device once implanted.

Document US20140012368 shows devices and methods for improving the implantation, retractability, or repositioning of a device for positioning a valve in a partition. The embodiments of the devices include pivotable sections which provide the ability to maintain the engagement of the device with a release system during implantation, in which the release system approaches an opening of the partition. The embodiments of the devices include configurations which allow for better recovery in a delivery system if a malfunction or problem with the patient's physiology is detected. This device is used to implant a flow control valve. These solutions are devices for treating heart failure. In particular, it is intended to create interatrial pressure outlets, shunts, and the like, which reduce the high pressure on one side of the heart, thus mitigating the resulting symptoms. Therefore, this solution aims to create an opening in the partition. This flow control element is a tissue valve such as a tricuspid valve, a bicuspid valve or a single-leaflet valve made of pericardial tissue from cattle, pigs, sheep or other animals, and therefore it has cusp-like foils made of preferably natural tissue and cusp-shaped.

Document US20160296684 shows endoluminal devices for the treatment of heart failure, which include a central body adapted to allow the passage of interatrial blood, an anchor fixed to the central body adapted to keep the endoluminal device in place inside a defect present in the atrial partition of a patient's heart, and a control element rotationally engaged with the central body. The rotation of the control element in relation to the central body creates a bidirectional flow of interatrial blood which allows to reduce the high pressure in the right and left parts of the heart. This solution has a valve which, depending on the rotating position of a diaphragm, which position has been previously fixed, allows a flow of blood. This valve is made with a central body shutter, the rigidity of which allows the central body to maintain a certain radial force outward, against the walls of the partition, thus reducing the risk of displacement or migration of the endoluminal device.

These solutions, although being advantageous in some aspects, are very complex to implement, and especially do not allow to substantially completely occlude the partition defect while choosing freely the crossing point or re-crossing point in different positions of the diaphragm, thus facilitating the approach of the surgical instrument to the surgery area which can be located very differently from patient to patient and from disease to disease.

Therefore, the need for an occluding device which is simple to manufacture, easy to apply, and flexible to use after implantation for crossing the defect or the now-occluded opening of the partition in case of subsequent surgery is still strongly felt.

### . Solution

Therefore, it is the object of the present invention to provide a crossable interseptal occluder device, having structural and functional features such as to meet the aforementioned needs and overcome the drawbacks mentioned above with reference to the devices of the prior art.

These and other objects are achieved by a device according to claim **1.**

Some advantageous embodiments are the subject of the dependent claims.

### . Drawings

. Further features and advantages of the invention will become apparent from the description provided below of preferred exemplary embodiment thereof, given by way of non-limiting example, with reference to the accompanying drawings, in which:
- Figure 1 is a diagrammatic sectional view of a heart in which defects or openings are present in the atrial and ventricular partitions;

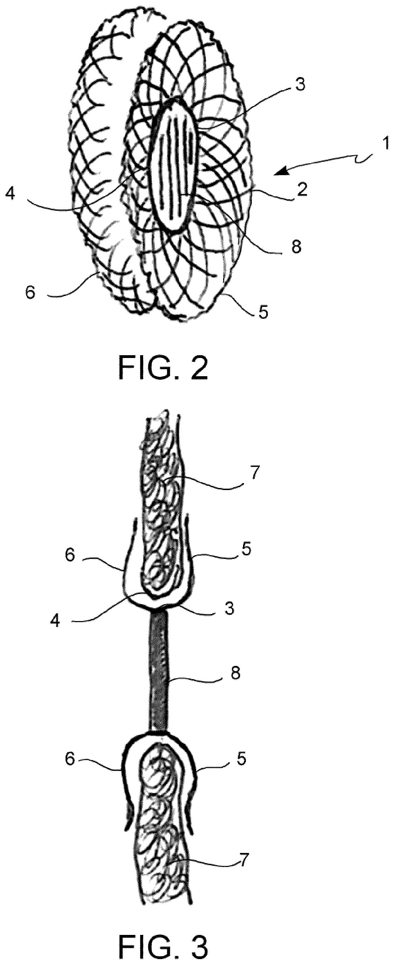

- Figure 2 shows an axonometric view of a crossable interseptal occluding device according to the present invention;
- Figure 3 is a cross-sectional view of a crossable interseptal occluding device applied to close a defect or opening of a partition;
- Figure 4A is a diagrammatic front view of only the part of the diaphragm of a crossable interseptal occluding device, made according to an embodiment with parallel fringes;
- Figure 4B is a diagrammatic front view of only the part of the diaphragm of a crossable interseptal occluding device, made according to an embodiment with radial fringes; this embodiment is not part of the present invention;
- Figure 5 is a diagrammatic front view of only the part of the diaphragm of a crossable interseptal occluding device, made according to an embodiment where two diaphragms at least partially overlap with each other and the fringes thereof, arranged parallel in the same diaphragm, are oriented orthogonal to the facing diaphragm;
- Figure 6 is a perspective view of only the part of the diaphragm of a crossable interseptal occluding device crossed by a medical device, here depicted as a tubular body only, by moving away and extending the fringes of said diaphragm;
- Figures 7A to 7E depict only the part of the diaphragm of a crossable interseptal occluding device, in a front view and in a local cross section of the fringes alone in order to show the shape of the sections of the fringes according to four different embodiments;
- Figures 8A and 8B show only the part of the diaphragm of a crossable interseptal occluding device, in a front view and in a local cross section of the fringes alone according to an embodiment with tapered fringes;
- Figure 9 depicts only the local cross sections of two diaphragms with tapered fringes, in a facing phase, in which one diaphragm is rotated and offset from the second one, so as to arrange the tapered fringes thereof between the tapered fringes of the facing diaphragm and mutually partially interpenetrated;

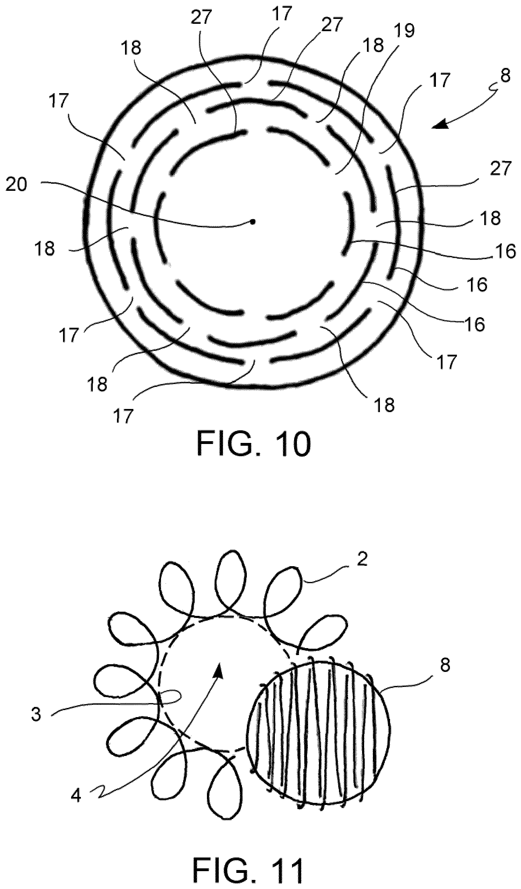

- Figure 10 is a diagrammatic front view of only the part of the diaphragm of a crossable interseptal occluding device, made according to an embodiment with radial fringes and annular path interrupted by small bridges; this embodiment is not part of the present invention;
- Figure 11 is a diagrammatic front view of an operation for connecting a diaphragm to a support structure to obtain a crossable interseptal occluding device;
- Figure 12 depicts a diaphragm of a crossable interseptal occluding device made with a single wire folded and forming wire stretches which are mutually substantially parallel;
- Figure 13 is a diagrammatic front view of only the part of the diaphragm of a crossable interseptal occluding device, made according to an embodiment where two diaphragms at least partially overlap with each other and the fringes thereof, shaped as wires, are arranged parallel in the same diaphragm, but are oriented orthogonal to the facing diaphragm;

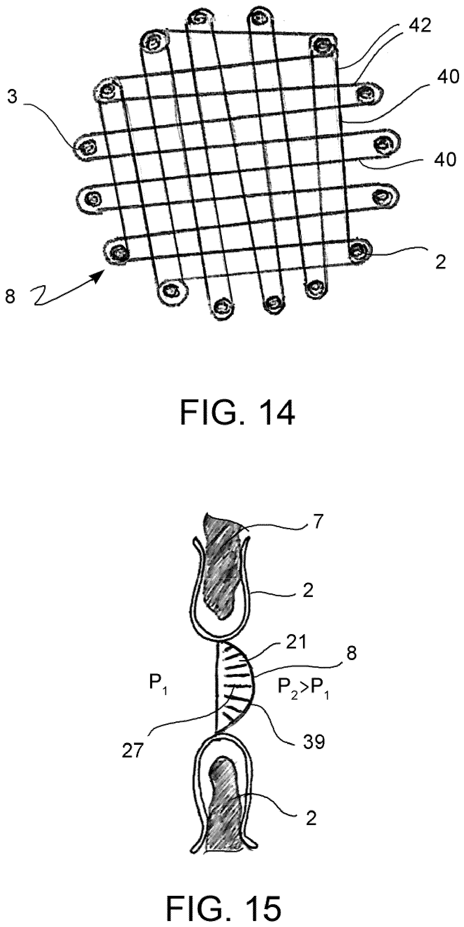

- Figure 14 depicts a diaphragm of a crossable interseptal occluding device made with wires folded and forming first wire stretches which are mutually substantially parallel and second stretches which are substantially parallel but transverse to the first stretches;
- Figure 15 is a cross-sectional view of a crossable interseptal occluding device applied to close a defect or opening of a partition, in which the diaphragm is dome-shaped with concavity facing the cavity or compartment where a higher pressure is expected; this embodiment is not part of the present invention;

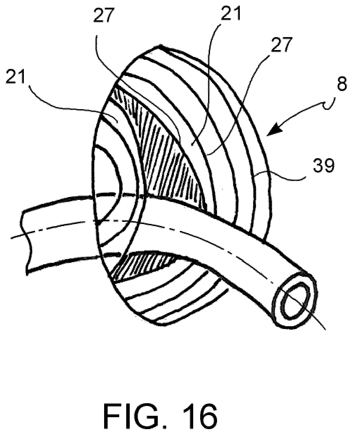

- Figure 16 is an axonometric view of a diaphragm of a crossable interseptal occluding device, in which the diaphragm is dome-shaped with concavity facing the cavity or compartment where a higher pressure is expected and is crossed by a medical device, here depicted in a tubular shape only, which moves and extends the fringes; this embodiment is not part of the present invention;

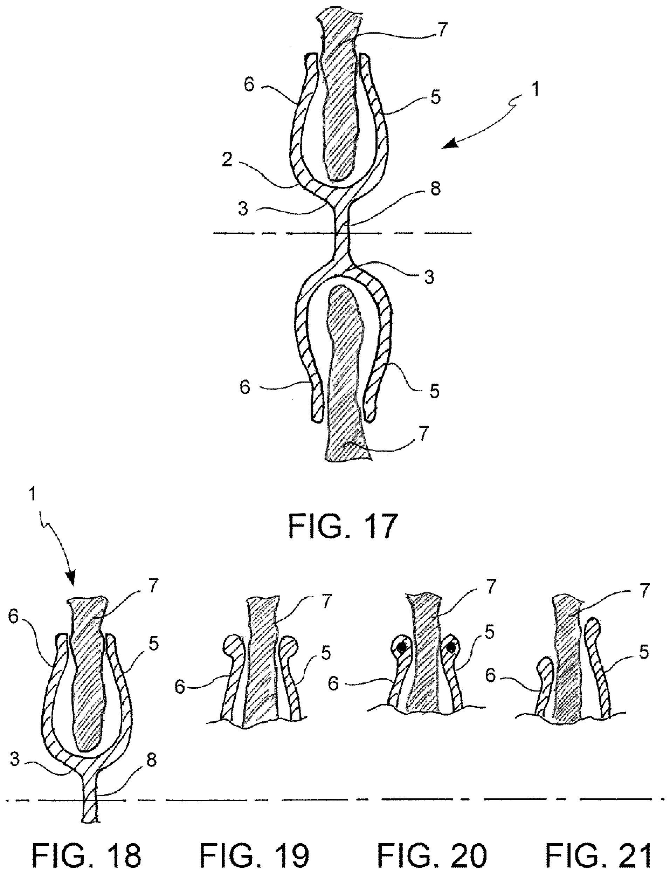

- Figure 17 shows a cross-sectional views of a crossable interseptal occluding device applied to close a defect or opening of a partition, in which the diaphragm is in one piece with the support structure anchored to the partition;
- Figures 18 to 21 show cross-sectional views of different embodiments of the anchoring portions of a support structure applied to a defect or opening of a partition and made in a single piece with the diaphragm;
- Figures 22 to 24 show a front view of different embodiments of anchoring portions of support structures of crossable interseptal occluding devices, in particular disc-shaped, three-lobed and angularly offset, six-lobed and angularly offset;
- Figure 25 is a front view of a crossable interseptal occluding device in which at least one of the anchoring portions of a support structure has circumferentially distributed openings;
- Figure 26 shows a diagrammatic front view of only the part of the diaphragm of a crossable interseptal occluding device, made according to an embodiment with fringes and wavy fringe edges or fringes with wavy fringe body, which is suitable for a greater elastic extension in case of re-crossing by a medical device;
- Figure 27 is a diagrammatic axonometric view of only the diaphragm of the crossable interseptal occluding device, in which the occluding bridges are according to a further embodiment;
- Figure 28 shows an axonometric sectional view according to line XXVII-XXVII in figure 27 in which the shapes of the cross sections of the occluding bridges are highlighted, showing a meshing or geometric coupling between the different elongated bodies of the occluding bridges, thus creating slits or cuts for separating the bodies of the bridges with a winding shape;
- Figure 29 is a front view of a diaphragm of a crossable interseptal occluding device, according to a further embodiment, where the occluding bridges have independent leaves in the central body portion thereof and inclined with respect to the plane of the diaphragm or lumen, so as to create winding path separation slits, for example by partially overlapping a bridge with the adjacent one;
- Figure 30 shows a section ac-cording to line XXX-XXX in Figure 29 of the diaphragm in Figure 29;

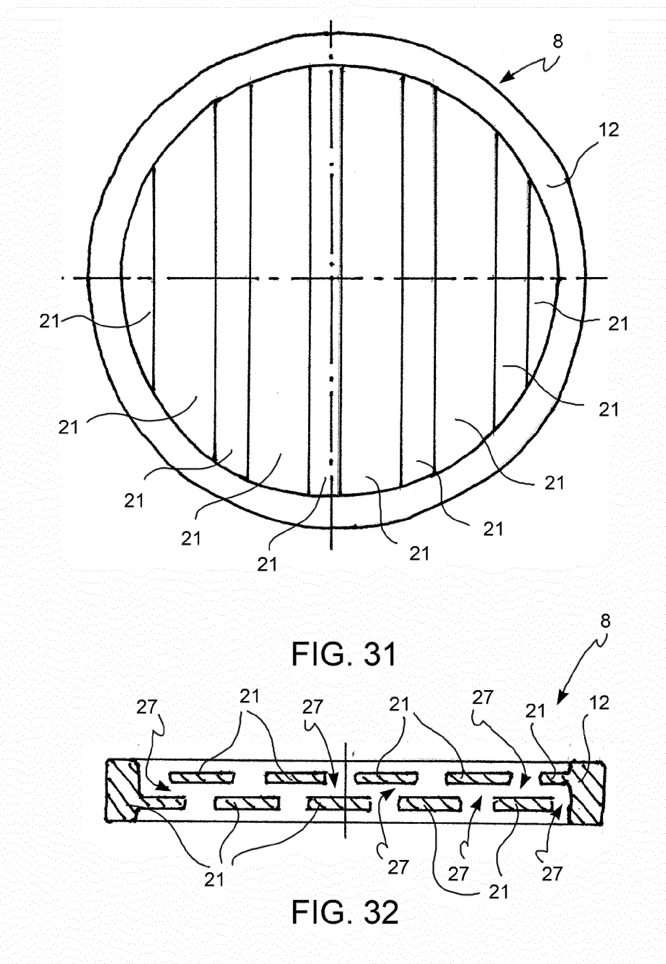

- Figure 31 is a front view of a diaphragm of a crossable interseptal occluding device, ac-cording to a further embodiment, where the occluding bridges have independent leaves in the central body portion thereof, which are arranged so as to be offset on two planes, alternatively on a first plane and on a second plane which is offset from and parallel to the first one, so as to create winding path separation slits, for example by partially overlapping a bridge with the adjacent one;
- Figure 32 shows a section ac-cording to line XXXII-XXXII in Figure 31 of the diaphragm in Figure 31;

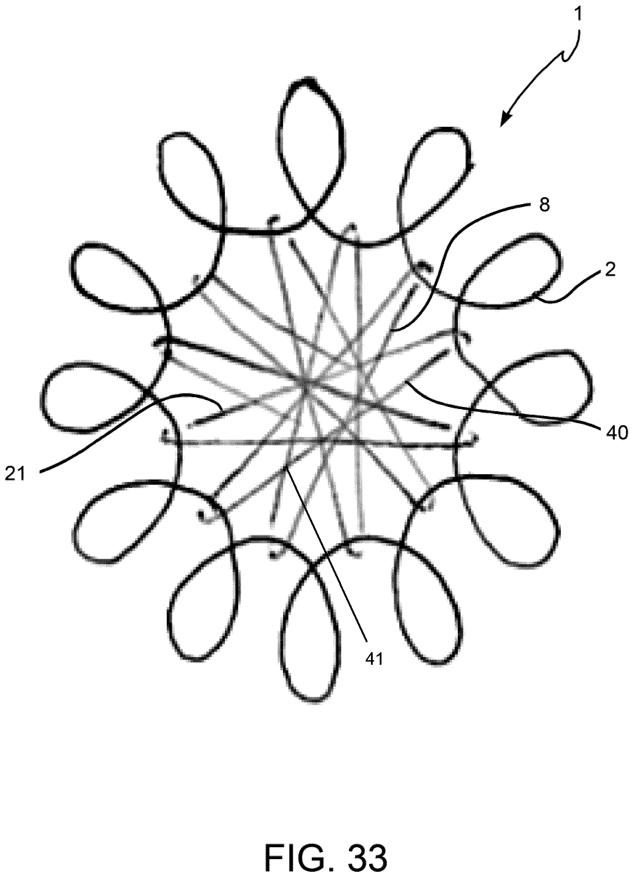

- Figure 33 is a diagrammatic view of a crossable interseptal occluding device in which the diaphragm is made from elements or a single thread-like element wrapped around the support structure, such as a tube, or an elastic thread, arranged so as to causally cross the area of the lumen and create a substantial occlusion in the majority of the lumen. This embodiment is not part of the present invention.

### . Description of some preferred embodiments

In accordance with a general embodiment, there is provided a crossable interseptal occluder device 1 comprising a support structure 2 in which said support structure 2 comprises a central support structure portion 3 which delimits a lumen 4.

Said support structure 2 comprises a first anchoring portion 5 and an opposite second anchoring portion 6.

Said support structure 2 being configured to expand and contract between a compressed tubular configuration for the insertion through the patient's vasculature and an expanded or extended configuration in which the first and second anchoring portions 5, 6 extend radially outwards from said central support structure portion 3 to compress a partition 7 therebetween by arranging said support structure 2 astride said partition 7 through a defect or hole or opening present in the partition 7.

Said crossable interseptal occluder device 1 further comprises at least one diaphragm 8.

Said diaphragm 8 being supported by said support structure 2 and arranged to close the majority of said lumen 4, when said diaphragm 8 is in a relaxed configuration with the supporting structure 2 being extended.

Said diaphragm 8 being configured to allow a medical instrument inserted into a first compartment 9 or 10 delimited by said septum 7 to pass through the diaphragm 8 and then through said lumen 4 entering a second compartment 10 or 9 delimited by said septum 7.

Said diaphragm 8 comprises a diaphragm edge 11 placed close to said central support structure portion 3 of said support structure 2.

Said diaphragm 8 comprises a plurality of elongated membrane occluding fringes or bridges 21 or occluding bridges 21.

Each of said occluding bridges 21 comprises an elongated body 50 having opposite elongated body ends or ends 51, 52 and opposite longitudinal edges or sides 22.

In a relaxed configuration of said diaphragm 8, each occluding bridge 21 of said plurality of occluding bridges 21 is arranged close to at least a further occluding bridge 21 of said plurality of occluding bridges 21.

At least one portion of said longitudinal sides 22 of each occluding bridge 21 and at least one portion of said longitudinal sides 22 of said at least a further occluding bridge 21 delimit at least one slit or one cut 27 into said diaphragm 8.

Both said opposite ends 51, 52 of each occluding bridge 21 are directly or indirectly connected to or supported by said support structure 2.

The term "occlude" means the possibility of minimizing or completely avoiding the flow of blood passing through said diaphragm. This definition is to describe an occlusion created by a plurality of occluding elements which, arranged to cover the lumen, create barriers while remaining at least partially independent of each other to be movable when necessary, but which tend to return, when not urged, to the occlusion position.

The term "relaxed position or configuration of the diaphragm" means a configuration both unimplanted and implanted in the patient in which the support structure is expanded or deployed or extended in which the diaphragm is not urged by external forces, but not necessarily without internal actions, such as a tension which tends to keep the occluding bridges in the occlusion position. For example, this position coincides with that of the support structure in an extended position, adapted to grasp the edges of the partition close to the edge of the lumen to be occluded. For example, but not necessarily, the relaxed position of the diaphragm coincides with an arrangement of the occluding bridges which is planar as a whole or on planes.

In accordance with an embodiment, in a relaxed configuration of said diaphragm 8, each of said longitudinal sides 22 delimits, with the side of an adjacent occluding bridge 21, an elongated and narrow opening or elongated slit 27.

In accordance with an embodiment, in a relaxed configuration of said diaphragm 8, each bridge of said plurality of occluding bridges 21 of each diaphragm 8 of said at least one diaphragm 8 lies in a single plane.

In accordance with an embodiment, in a relaxed configuration of said diaphragm 8, said plurality of occluding bridges 21 is arranged with the bridges placed side by side.

In accordance with an embodiment, in a relaxed configuration of said diaphragm 8, each occluding bridge 21 is arranged close to at least a further occluding bridge 21 facing at least one portion of a longitudinal side 22 thereof to at least one portion of a longitudinal side 822 of said further occluding bridge 21.

In accordance with an embodiment, in a relaxed configuration of said diaphragm 8, said slit or cut 27 is one elongated slit 27; and/or wherein said slit or cut 27 is a long and narrow opening 11 delimited by occluding bridges 21.

In accordance with an embodiment, in a relaxed configuration of said diaphragm 8, said each occluding bridge 21 is placed adjacent to said at least a further occluding bridge 21.

In accordance with an embodiment, in a relaxed configuration of said diaphragm 8, each of said opposite longitudinal sides 22 lies so that the sum of the elongated bodies 50 of said occluding bridges 21 occludes the majority of said lumen 4.

In accordance with an embodiment, in a relaxed configuration of said diaphragm 8, said each occluding bridge 21 is arranged parallel to said at least a further occluding bridge 21.

In accordance with an embodiment, in a relaxed configuration of said diaphragm 8, said slit or cut 27 has a longitudinal extension equal to the entire longitudinal extension of said elongated body 50 of said each occluding bridge 21 which delimits said slit or cut 27.

In accordance with an embodiment, in a relaxed configuration of said diaphragm 8, all slits or cuts 27 are arranged parallel to one another.

In accordance with an embodiment, except for the opposite elongated body ends 51, 52, each elongated body 50 of each occluding bridge 21 is independent of other elongated bodies 50 of other occluding bridges 21.

In accordance with an embodiment not part of the present invention, in a relaxed configuration of said diaphragm 8, the occluding bridges 21 of said plurality of occluding bridges 21 are randomly distributed in order to occlude the majority of said lumen 4.

In accordance with an embodiment, in a relaxed configuration of said diaphragm 8, the occluding bridges 21 of said plurality of occluding bridges 21 are arranged so as to avoid from intertwining or interlacing with one another.

In accordance with an embodiment, in a relaxed configuration of said diaphragm 8, each of said bridges of said plurality of occluding bridges 21 lies in a plane arranged orthogonal to an axis of said lumen 4.

In accordance with an embodiment, in a relaxed configuration of said diaphragm 8, all bridges of said plurality of occluding bridges 21 of each diaphragm 8 lie in the same plane.

In accordance with an embodiment, both said opposite ends 51, 52 of each occluding bridge 21 are directly or indirectly connected to or supported by said central support structure portion 3 of said support structure 2.

In accordance with an embodiment, said opposite longitudinal sides 22 of said plurality of occluding bridges 21 are parallel to one another.

In accordance with an embodiment, said plurality of occluding bridges 21 comprises opposite longitudinal sides 22 and where said opposite longitudinal sides 22 of adjacent occluding bridges 21 are arranged side by side and in contact with one another.

In accordance with an embodiment, said diaphragm 8 is made of an elastic material, for example silicone or medical elastomer or polyurethane or bioerodible or bioabsorbable material.

In accordance with an embodiment, said diaphragm 8 is made of an elastic material capable of deformation at least between 10% and 20%.

In accordance with an embodiment, said diaphragm 8 is made of an elastic material, for example silicone or medical elastomer or polyurethane or bioerodible or bioabsorbable material; and where said diaphragm 8 is made of an elastic material capable of deformation of at least 150%.

In accordance with an embodiment, said diaphragm 8 is an elastic membrane.

In accordance with an embodiment, said occluding bridges 21 of said diaphragm 8 are separated from one another in the extension thereof or central occluding bridge portion 29 but joined together in a single piece.

In accordance with an embodiment, said diaphragm edge 11 and said occluding bridges 21 are in a single piece.

In accordance with an embodiment, at least one of said occluding bridges 21 has a longitudinal extension and comprises an occluding bridge section 23 evaluated in a transverse direction to said longitudinal direction of circular shape.

In accordance with an embodiment, at least one of said occluding bridges 21 has a longitudinal extension and comprises an occluding bridge section 23 evaluated in a transverse direction to said longitudinal direction of square or rectangular shape.

In accordance with an embodiment, at least one of said occluding bridges 21 has a longitudinal extension and comprises an occluding bridge section 23 evaluated in a transverse direction to said longitudinal direction of tapered shape passing from the center of said section to a section end 24 thereof.

In accordance with an embodiment, at least one of said occluding bridges 21 has a longitudinal extension and comprises an occluding bridge section 23 evaluated in a transverse direction to said longitudinal direction of tapered shape passing from a first section base side 25 to a section end 24 thereof.

In accordance with an embodiment, at least one of said occluding bridges 21 has a longitudinal extension and comprises an occluding bridge section 23 evaluated in a transverse direction to said longitudinal direction of tapered shape passing from a first section base side 25 to smaller section side 26.

In accordance with an embodiment, at least one of said occluding bridges 21 has a longitudinal extension and comprises an occluding bridge section 23 evaluated in a transverse direction to said longitudinal direction of oval or triangular or rhomboidal or trapezoidal shape.

In accordance with an embodiment, at least one pair of said occluding bridges 21 has a longitudinal extension and comprises an occluding bridge section 23 evaluated in a transverse direction to said longitudinal direction of concave shape, in the first occluding bridge 21 of said pair, facing a convex shape, in the second occluding bridge 21 of said pair, and said concave and convex shapes are at least partially meshed with each other.

In accordance with an embodiment, at least one pair of said occluding bridges 21 has a longitudinal extension and comprises an occluding bridge section 23 evaluated in a transverse direction to said longitudinal direction of concave shape, in the first occluding bridge 21 of said pair, facing a convex shape, in the second occluding bridge 21 of said pair, and said concave and convex shapes are at least partially meshed with each other and said shapes are arched shapes.

In accordance with an embodiment, at least one pair of said occluding bridges 21 has a longitudinal extension and comprises an occluding bridge section 23 evaluated in a transverse direction to said longitudinal direction of concave shape, in the first occluding bridge 21 of said pair, facing a convex shape, in the second occluding bridge 21 of said pair, and said concave and convex shapes are at least partially meshed with each other and said shapes are triangular or trapezoidal shapes.

In accordance with an embodiment, a pair of diaphragms 28 is included, each of said diaphragms 8 of said pair of diaphragms 28 comprising a plurality of occluding bridges 21.

Said diaphragms 8 of said pair of diaphragms 28 are arranged facing each other and with the plurality of occluding bridges 21 of the first diaphragm 8 of said pair of diaphragms 28 being offset with respect to the plurality of diaphragm occluding bridges 21 of the second diaphragm 8 of said pair of diaphragms 28 by overlapping at least partially said plurality of occluding bridges 21 of the first diaphragm 8 to said plurality of elongated slits or cuts 27 of said second diaphragm 8.

In accordance with an embodiment, said plurality of occluding bridges 21 of the first diaphragm 8 has tapered occluding bridge sections 23, for example triangular or rhomboidal in shape.

The tapering of the occluding bridge sections 23 of said plurality of occluding bridges 21 of the first diaphragm 8 tapers towards said second diaphragm 8.

In accordance with an embodiment, said plurality of occluding bridges 21 of the first diaphragm 8 interpenetrates at least partially between said occluding bridges 21 of said plurality of occluding bridges 21 of the second diaphragm 8.

In accordance with an embodiment, said diaphragm 8 has a concave body or concavity facing one of said first or second compartment 9, 10.

In accordance with an embodiment not part of the present invention, said diaphragm 8 is dome-shaped.

In accordance with an embodiment, said diaphragm 8 comprises a diaphragm stiffening structure 30.

In accordance with an embodiment, said diaphragm 8 comprises a diaphragm stiffening structure 30, said diaphragm stiffening structure 30 comprises diaphragm ribs 31 adapted to create a support scaffold.

In accordance with an embodiment, said diaphragm 8 comprises a diaphragm stiffening structure 30, said diaphragm stiffening structure 30 comprises diaphragm ribs 31 in a single piece with said diaphragm 8.

In accordance with an embodiment, said diaphragm 8 comprises a diaphragm stiffening structure 30, said diaphragm stiffening structure 30 comprises at least one diametrical rib 32 extending along a diameter of said diaphragm 8.

In accordance with an embodiment, said diaphragm 8 comprises a diaphragm stiffening structure 30, said diaphragm stiffening structure 30 comprises at least one circumferentially extending rib 33.

In accordance with an embodiment, said diaphragm 8 comprises a diaphragm stiffening structure 30, said diaphragm stiffening structure 30 comprises at least one radially extending rib 34.

In accordance with an embodiment, said diaphragm 8 comprises a diaphragm stiffening structure 30, said diaphragm stiffening structure 30 comprises at least one rib 35 extending along a circle chord.

In accordance with an embodiment, said diaphragm 8 comprises a diaphragm stiffening structure 30, said diaphragm stiffening structure 30 comprises diaphragm ribs 31 adapted to create a support frame and said at least one rib is of elastic and/or extensible shape or material.

In accordance with an embodiment, said diaphragm 8 comprises a first diaphragm portion 12 having an external continuous annular portion directly or indirectly connected to said support structure 2.

Each occluding bridge end 51, 52 is connected to said external annular diaphragm portion 12.

In accordance with an embodiment, said diaphragm 8 comprises an external annular diaphragm portion 12 directly or indirectly connected to said support structure 2.

Each occluding bridge end 51, 52 is in a single piece with said external annular diaphragm portion 12.

In accordance with an embodiment not forming part of the present invention, said diaphragm 8 comprises an external annular diaphragm portion 12 directly or indirectly connected to said support structure 2.

Said diaphragm 8 comprises a central disc-shaped diaphragm portion 20.

Said plurality of occluding bridges 21 is arranged radially with an external end 51 connected to said external annular diaphragm portion 12 and the internal end 52 connected to said central diaphragm disc 20.

In accordance with an embodiment, said diaphragm 8 comprises an external annular diaphragm portion 12 directly or indirectly connected to said support structure 2.

Said diaphragm 8 comprises a central disc-shaped diaphragm portion 20.

Said plurality of occluding bridges 21 is arranged radially with an external end 51 connected to said external annular diaphragm portion 12 and the internal end 52 connected to said central diaphragm disc 20.

The opposite occluding bridge edges or sides 22 converge towards said central diaphragm disc 20; and where each occluding bridge edge or side 22 is arranged parallel to the adjacent occluding bridge edge or side 22 of the adjacent occluding bridge 21.

In accordance with an embodiment, said diaphragm 8 comprises a plurality of hollow tubes or wires or elastic threads 42 comprising hollow tube or elastic thread stretches 41 which form said diaphragm 8.

In accordance with an embodiment, said diaphragm 8 comprises a single hollow tube or wire or elastic thread 40 folded to form hollow tube or wire or elastic thread stretches 41 which form said diaphragm 8.

In accordance with an embodiment, said plurality of hollow tubes or wires or elastic threads 42 or said single folded hollow tube or wire or elastic thread 40 is connected or folded around said support structure 2.

In accordance with an embodiment, said plurality of hollow tubes or wires or elastic threads 42 or said single folded hollow tube or wire or elastic thread 40 is connected or folded around said central support structure portion 3.

In accordance with an embodiment, said diaphragm 8 comprises a plurality of hollow tube or wire or elastic thread stretches 41 which are parallel to one another.

In accordance with an embodiment, said diaphragm 8 comprises a plurality of hollow tube or wire or elastic thread stretches 41 which are mutually arranged side by side.

In accordance with an embodiment, said diaphragm 8 comprises a plurality of hollow tube or wire or elastic thread stretches 41 which are arranged adjacent to one another.

In accordance with an embodiment, said diaphragm 8 comprises a plurality of radially arranged hollow tube or wire or elastic thread stretches 41.

In accordance with an embodiment, said diaphragm 8 comprises a first plurality of hollow tube or wire or elastic thread stretches 41 which are parallel to one another and a second plurality of hollow tube or wire or elastic thread stretches 41 which, in said second plurality, are parallel to one another but orthogonal to said first plurality of hollow tube or wire or elastic thread stretches 41.

In accordance with an embodiment, said diaphragm 8 comprises a first plurality of hollow tube or wire or elastic thread stretches 41 which are parallel to one another and at least a second plurality of hollow tube or wire or elastic thread stretches 41 which, in said at least a second plurality, are parallel to one another but inclined with respect to said first plurality of hollow tube or wire or elastic thread stretches 41.

In accordance with an embodiment, said plurality of hollow tubes or wires or elastic threads 42 or said single hollow tube or wire or elastic thread 40 is made of a superelastic material, preferably Nitinol, or silicone, or polyurethane or medical elastomer or of a bioerodable material.

In accordance with an embodiment, said device comprises at least two diaphragms 8.

Said at least two diaphragms 8 at least partially overlap with each other.

In accordance with an embodiment, said device comprises at least two diaphragms 8.

Said at least two diaphragms 8 at least partially overlap with each other so as to at least partially face at least one occluding bridge 21 of a first diaphragm 8 to at least one elongated and narrow opening or slit or cut 27 of a second diaphragm 8.

In accordance with an embodiment, said device comprises at least two diaphragms 8.

Said at least two diaphragms 8 at least partially overlap with each other so as to at least partially enter an elongated and narrow opening or slit or cut 27 of a second diaphragm 8 with at least one occluding bridge 21 of a first diaphragm 8, by arranging itself between two adjacent occluding bridges 21 of said second diaphragm 8.

In accordance with an embodiment, said support structure 2 is in a single piece. In accordance with an embodiment, said support structure 2 and said diaphragm 8 are in a single piece.

In accordance with an embodiment, said support structure 2 is made of medical elastomer.

In accordance with an embodiment, two diaphragms 8 are included, and where said two diaphragms 8 angularly overlap with each other by arranging the occluding bridges 21 of a first diaphragm 8 crossed with respect to the occluding bridges 21 of the other diaphragm 8.

In accordance with an embodiment, two diaphragms 8 are included; and where said two diaphragms 8 overlap with each other, the occluding bridges 21 of a first diaphragm 8 are arranged inclined with respect to the occluding bridges 21 of the second diaphragm 8.

In accordance with an embodiment, two diaphragms 8 are included; and where said two diaphragms 8 angularly overlap with each other by arranging the occluding bridges 21 of a first diaphragm 8 so as to be orthogonal to the occluding bridges 21 of the other diaphragm 8.

In accordance with an embodiment, two diaphragms 8 are included; and where said two diaphragms 8 angularly overlap with each other by arranging the occluding bridges 21 of a first diaphragm 8 at an angle between 20 degrees and 90 degrees with respect to the occluding bridges 21 of the other diaphragm 8.

In accordance with an embodiment, said device 1 comprises a diaphragm 8 with occluding bridges 21 in a thread; said occluding bridges 21 are mounted to frame and said frame is fixed to said support structure 2.

In accordance with an embodiment, said device 1 comprises a diaphragm 8 with occluding bridges 21 having a wavy occluding bridge body 50.

In accordance with an embodiment, said device 1 comprises a diaphragm 8 with occluding bridges 21 having a wavy occluding bridge body 50, thus allowing each wavy bridge 21 urged by a possible crossing of the diaphragm itself, for example by a medical device, to elongate, and then return to a closed position of the diaphragm 8.

In accordance with an embodiment, said device 1 comprises a diaphragm 8 with occluding bridges 21 having wavy occluding bridge edges 22, the wavy bridge edge being the side portion of the occluding bridge 21 which delimits said elongated and narrow opening or slit or cut 27, thus allowing each occluding bridge urged by a possible crossing of the diaphragm by a medical device, to elongate, and facilitating, once the device has been removed, the re-closing of the wavy bridge 21 and therefore of the diaphragm 8 itself.

In accordance with an embodiment, when under relaxed conditions, said diaphragm 8 is planar in shape and said occluding bridges 21 are tensioned on said support structure 2.

In accordance with an embodiment, said occluding bridges 21 are slats or strips or foils or wires or hollow tubes.

In accordance with an embodiment, under a relaxed condition of said diaphragm 8, said occluding bridges 21 are separated by slits or cuts 27 in order to avoid material continuity between one bridge and the other.

In accordance with an embodiment, under a relaxed condition of said diaphragm 8 and when the device is not implanted in a human body, said occluding bridges 21 close the majority of said lumen 4.

In accordance with an embodiment, under a relaxed condition of said diaphragm 8, said occluding bridges 21 cover 70-100% of the lumen 4.

In accordance with an embodiment, under a relaxed condition of said diaphragm 8, said occluding bridges 21 are uniformly distributed over the lumen 4.

In accordance with an embodiment, under a relaxed condition of said diaphragm 8, said occluding bridges 21 delimit slits or cuts 27 which leave free passages among said occluding bridges 21 of no more than 25% of said lumen 4.

In accordance with an embodiment, said diaphragm 8 is obtained from a single sheet where said elongated slits 27 are made by laser cutting.

In accordance with an embodiment, said diaphragm 8 is obtained from a single sheet 53 in which said elongated slits 27 are made by cuts.

Said sheet 53 is fixed to the support structure 2 crossing said lumen 4 twice.

In accordance with an embodiment, said occluding bridges 21 are concentric rings which extend along an annular path 16.

Adjacent concentric occluding bridges 21 are connected by means of radial small bridges or cut interruptions 17, 18, 19.

Said radial small bridges or cut interruptions 17, 18, 19 are circumferentially offset between three near concentric occluding bridges 21 so as to avoid a radial alignment of a plurality of radial small bridges or cut interruptions.

The sides 22 of adjacent concentric occluding bridges 21 delimit arched slits or cuts 27 aligned along a circumferential path interrupted by said radial small bridges or cut interruptions 17, 18, 19.

In accordance with an embodiment, said diaphragm 8 comprises an external continuous annular diaphragm portion 12 directly or indirectly connected to said support structure 2.

The radially outermost occluding bridge 21 is connected to said external continuous annular diaphragm portion 12 via said radial small bridges or cut interruptions 17, 18, 19.

In accordance with a general embodiment, a crossable interseptal occluder device 1 comprises a support structure 2.

Said support structure 2 comprises a central support structure portion 3 which delimits a lumen 4.

Said support structure 2 comprises a first anchoring portion 5 and an opposite second anchoring portion 6.

Said support structure 2 is configured to expand and contract between a compressed tubular configuration for the insertion through the patient's vasculature and an expanded configuration in which the first and second anchoring portions 5, 6 extend radially outwards from said central support structure portion 3 to compress a partition 7 therebetween by arranging said support structure 2 astride said partition 7 through a defect or hole or opening present in the partition 7.

Said crossable interseptal occluder device 1 further comprises a diaphragm 8.

Said diaphragm 8 is supported by said support structure 2 and arranged to close at least partially said lumen 4.

Said diaphragm 8 being configured to allow a medical instrument inserted into a first compartment 9 or 10 delimited by said septum 7 to pass through the diaphragm 8 and then through said lumen 4 entering a second compartment 10 or 9 delimited by said septum 7.

Said diaphragm 8 comprises a diaphragm edge 11 placed close to said central support structure portion 3 of said support structure 2.

Advantageously, said diaphragm 8 comprises at least one elongated and narrow opening 27.

The term "elongated and narrow opening" means a longitudinally extending opening, predominantly with respect to the extension thereof which is transverse to said longitudinal direction.

Alternatively, said elongated and narrow opening 27 comprises one of the following embodiments:
- it extends from a first diaphragm portion 12 of said diaphragm 8 placed close to a first edge stretch 13 of said diaphragm edge 11, to a second diaphragm portion 14 of said diaphragm 8 placed close to a second edge stretch 15 of said diaphragm edge 11;
   or
- it extends from a first diaphragm portion 12 of said diaphragm 8 placed close to a first edge stretch 13 of said diaphragm edge 11, to a second diaphragm portion 14 of said diaphragm 8 placed close to a diaphragm center 36 of said diaphragm 8;
   or
- it extends close to said diaphragm edge 11 to cover an annular path 16 interrupted by small diaphragm bridges 17, 18, 19 which connect a central diaphragm portion 20 to said diaphragm edge 11 by forming a plurality of elongated and narrow openings 11 placed one after the other.

In accordance with an alternative embodiment, said at least one elongated and narrow opening 27 is a plurality of long and narrow openings 11 delimited by a plurality of elongated membrane fringes 21, or membrane fringes 21 or occluding bridges 21.

In accordance with an alternative embodiment, in said diaphragm 11 said multiple membrane fringes 21 are parallel to one another.

In accordance with an alternative embodiment, in said diaphragm 11 said multiple membrane fringes 21 are mutually placed side by side and spaced apart from said plurality of long and narrow openings 11.

In accordance with an alternative embodiment, said plurality of membrane fringes 21 comprises membrane fringe edges 22 and where the membrane fringe edges 22 of adjacent membrane fringes 21 are mutually placed side by side and in contact with one other so that said elongated and narrow opening 27 is a slit.

In accordance with an alternative embodiment, said plurality of membrane fringes 21 comprises membrane fringe edges 22 and where the membrane fringe edges 22 of adjacent membrane fringes 21 are separated from one another so that said elongated and narrow opening 27 is a free opening.

In accordance with an alternative embodiment, said diaphragm 8 is made of an elastic material, for example silicone or medical elastomer or polyurethane or of a bioerodable material.

In accordance with an alternative embodiment, said diaphragm 8 is a membrane.

In accordance with an alternative embodiment, said diaphragm 8 is an elastic membrane.

In accordance with an alternative embodiment, said membrane fringes 21 of said diaphragm 8 are separated from one another in the extension thereof or central fringe portion 29 but joined together in a single piece.

In accordance with an alternative embodiment, said diaphragm edge 11 and said membrane fringes 21 are in a single piece.

In accordance with an alternative embodiment, each of said membrane fringes 21 has a longitudinal extension and comprises a fringe section 23 evaluated in a transverse direction to said longitudinal direction of square or rectangular shape.

In accordance with an alternative embodiment, each of said membrane fringes 21 has a longitudinal extension and comprises a fringe section 23 evaluated in a transverse direction to said longitudinal direction of tapered shape passing from the center of said section to a section end 24 thereof.

In accordance with an alternative embodiment, each of said membrane fringes 21 has a longitudinal extension and comprises a fringe section 23 evaluated in a transverse direction to said longitudinal direction of tapered shape passing from a first section base side 25 to a section end 24.

In accordance with an alternative embodiment, each of said membrane fringes 21 has a longitudinal extension and comprises a fringe section 23 evaluated in a transverse direction to said longitudinal direction of tapered shape passing from a first section base side 25 to a smaller section side 26.

In accordance with an alternative embodiment, each of said membrane fringes 21 has a longitudinal extension and comprises a fringe section 23 evaluated in a transverse direction to said longitudinal direction of oval or triangular or rhomboidal or trapezoidal shape.

In accordance with an alternative embodiment, a pair of diaphragms 28 is included, each of said diaphragms 8 of said pair of diaphragms 28 comprising a plurality of membrane fringes 21 which delimit a plurality of elongated and narrow openings 27.

Said diaphragms 8 of said pair of diaphragms 28 are arranged facing each other and with the plurality of membrane fringes 21 of the first diaphragm 8 of said pair of diaphragms 28 being offset with respect to the plurality of membrane fringes 21 of the second diaphragm 8 of said pair of diaphragms 28 by overlapping at least partially said plurality of membrane fringes 21 of the first diaphragm 8 to said plurality of elongated and narrow openings 27 of said second diaphragm 8.

In accordance with an alternative embodiment, said plurality of membrane fringes 21 of the first diaphragm 8 has tapered fringe sections 23.

In accordance with an alternative embodiment, said plurality of membrane fringes 21 of the first diaphragm 8 has tapered fringe sections 23, for example with rhomboid- or triangle-shaped section.

In accordance with an alternative embodiment, the tapering of the fringe sections 23 of said plurality of membrane fringes 21 of the first diaphragm 8 tapers towards said second diaphragm 8.

In accordance with an alternative embodiment, said plurality of membrane fringes 21 of the first diaphragm 8 interpenetrates at least partially between said membrane fringes 21 of said plurality of membrane fringes 21 of the second diaphragm 8.

In accordance with an alternative embodiment, said diaphragm 8 has a concave body or concavity facing one of said first or second compartment 9, 10.

In accordance with an alternative embodiment not part of the present invention, said diaphragm 8 is dome-shaped.

In accordance with an alternative embodiment, said diaphragm 8 comprises a diaphragm stiffening structure 30.

In accordance with an alternative embodiment, said diaphragm stiffening structure 30 comprises diaphragm ribs 31 adapted to create a support scaffold.

In accordance with an alternative embodiment, said diaphragm stiffening structure 30 comprises diaphragm ribs 31 in a single piece with said diaphragm 8.

In accordance with an alternative embodiment, said diaphragm stiffening structure 30 comprises at least one diametrical rib 32 extending along a diameter of said diaphragm 8.

In accordance with an alternative embodiment, said diaphragm stiffening structure 30 comprises at least one circumferentially extending rib 33.

In accordance with an alternative embodiment, said diaphragm stiffening structure 30 comprises at least one radially extending rib 34.

In accordance with an embodiment, said at least one radially extending rib is made of an extendable, i.e. stretchable, elastic material.

In accordance with an alternative embodiment, said diaphragm stiffening structure 30 comprises at least one rib 35 extending along a circle chord.

In accordance with an embodiment, said at least one rib extending along a circle chord is made of an extendable, i.e. stretchable, elastic material.

In accordance with an alternative embodiment not part of the present invention, said diaphragm 8 comprises at least one diaphragm portion shaped as a diaphragm half-dome 38, where said diaphragm half-dome 38 is separated from one diametrical rib 32 of one diaphragm stiffening structure 30 by an elongated and narrow opening 27 shaped as an arch.

In accordance with an alternative embodiment, said elongated and narrow opening 27 shaped as an arch extends from a first diaphragm portion 12 of said diaphragm 8 placed close to a first edge stretch 13 of said diaphragm edge 11, to a second diaphragm portion 14 of said diaphragm 8 placed close to a second edge stretch 15 of said diaphragm edge 11.

In accordance with an alternative embodiment not part of the present invention, said diaphragm 8 comprises two diaphragm portions shaped as a diaphragm half-dome 38, where said diaphragm half-domes 38 are mutually placed side by side to form a diaphragm dome 39 and each of said diaphragm half-domes 38 is separated from a diametrical rib 32 of a diaphragm stiffening structure 30 by an elongated and narrow opening 27 shaped as an arch.

In accordance with an alternative embodiment not part of the present invention, said elongated and narrow opening 27 shaped as an arch of each of said two diaphragm half-domes 38 extends from a first diaphragm portion 12 of said diaphragm 8 placed close to a first edge stretch 13 of said diaphragm edge 11, to a second diaphragm portion 14 of said diaphragm 8 placed close to a second edge stretch 15 of said diaphragm edge 11.

In accordance with an alternative embodiment, said diaphragm 8 comprises a plurality of elastic threads 42 comprising elastic thread stretches 41 which form said diaphragm 8.

In accordance with an alternative embodiment, said diaphragm 8 comprises a single elastic thread 40 folded to form elastic thread stretches 41 which form said diaphragm 8.

In accordance with an alternative embodiment, said plurality of elastic threads 42 or said single folded elastic thread 40 is connected or folded around said support structure 2.

In accordance with an alternative embodiment, said plurality of elastic threads 42 or said single folded elastic thread 40 is connected or folded around said central support structure portion 3.

In accordance with an alternative embodiment, said diaphragm 8 comprises a plurality of elastic thread stretches 41 which are parallel to one another.

In accordance with an alternative embodiment, said diaphragm 8 comprises a plurality of elastic thread stretches 41 which are mutually arranged side by side.

In accordance with an alternative embodiment, said diaphragm 8 comprises a plurality of elastic thread stretches 41 which are arranged adjacent to one another.

In accordance with an alternative embodiment, said diaphragm 8 comprises a plurality of radially arranged elastic thread stretches 41.

In accordance with an alternative embodiment, said diaphragm 8 comprises a first plurality of elastic thread stretches 41 which are parallel to one another and a second plurality of elastic thread stretches 41 which, in said second plurality, are parallel to one another but orthogonal to said first plurality of elastic thread stretches 41.

In accordance with an alternative embodiment, said diaphragm 8 comprises a first plurality of elastic thread stretches 41 which are parallel to one another and at least a second plurality of elastic thread stretches 41 which, in said at least a second plurality, are parallel to one another but inclined with respect to said first plurality of elastic thread stretches 41.

In accordance with an alternative embodiment, said plurality of elastic threads 42 or said single elastic thread 40 is made of a superelastic material, preferably Nitinol, or silicone, or polyurethane or medical elastomer or of a bioerodable material.

In accordance with an alternative embodiment, said device comprises at least two diaphragms 8.

In accordance with an alternative embodiment, said at least two diaphragms 8 at least partially overlap with each other.

In accordance with an alternative embodiment, said at least two diaphragms 8 at least partially overlap with each other so as to at least partially face a membrane fringe 21 of a first diaphragm 8 to an elongated and narrow opening 27 of a second diaphragm 8.

In accordance with an alternative embodiment, said at least two diaphragms 8 at least partially overlap with each other so as to at least partially enter an elongated and narrow opening 27 of a second diaphragm 8 with at least one membrane fringe 21 of a first diaphragm 8, by arranging itself between two adjacent membrane fringes 21 of said second diaphragm 8.

In accordance with an alternative embodiment, said support structure 2 is in a single piece.

In accordance with an alternative embodiment, said support structure 2 and said diaphragm 8 are in a single piece.

In accordance with an alternative embodiment, said support structure 2 is made of medical elastomer.

Those skilled in the art can make several changes and adaptations to the embodiments described above, and replace elements with others which are functionally equivalent, in order to meet contingent and specific needs, without however departing from the scope of the following claims.

Some exemplary embodiments of the present invention will be described below.

In accordance with an embodiment, the device 1 is made to be implanted by means of minimally invasive or percutaneous techniques.

One of the peculiarities of the device 1 consists in the possibility of being crossed again after some time from its implantation, in order to start new interseptal procedures.

In accordance with an embodiment, the architecture of the device 1 is based on two components or parts:
- a frame, or support structure 2, dedicated to the positioning and anchoring in situ of the device, generally toroidal in shape with opposing "umbrellas" or "donuts", positioned astride the defect or hole or opening, with the umbrellas or donuts arranged on opposite sides of the partition 100;
- a diaphragm 8, for example a central diaphragm, dedicated to the occlusion of the defect/hole, for example an iatrogenic or congenital defect/hole 107, with the possibility of being crossed again after some time.

The support structure 2 can be identified among those currently used or in any case described in the prior art, or it can be conceived and implemented according to an embodiment as previously described here.

From the construction point of view, for example, two main types of solutions can be referred to:
- support structure 2 obtained by means of techniques of braiding metal wires made of superelastic alloy (e.g. Nitinol; CoCr);
- support structure 2 obtained from a laser-cut and then shaped metal tube made of superelastic alloy.

For the diaphragm 8, or occluding diaphragm, some exemplary embodiments are described below.
1) Diaphragm 8 with occluding bridges in an "elastic fringe curtain".

This diaphragm 8 consists of thin strips (fringes 21) of elastic and highly extendable material. The fringes 21 are parallel and adjacent to one another, or with a small gap to space them apart, or arranged in a sunburst pattern, or according to other construction schemes. The diaphragm 8 can also consist of one or more layers of variously crossed fringes 21. Upon crossing by the catheter, the fringes 21 move apart and elongate, thus allowing the re-crossing. The re-crossing point can be freely decided by the operator at any point of the diaphragm 8. Advantageously, the suggested solution for diaphragm and fringes, after the removal of the device, allows to re-close the fringes 21 and therefore the diaphragm 8 itself.

### . 1-a) diaphragm with occluding bridges in an "elastic fringe curtain" in the shape of a thin disc

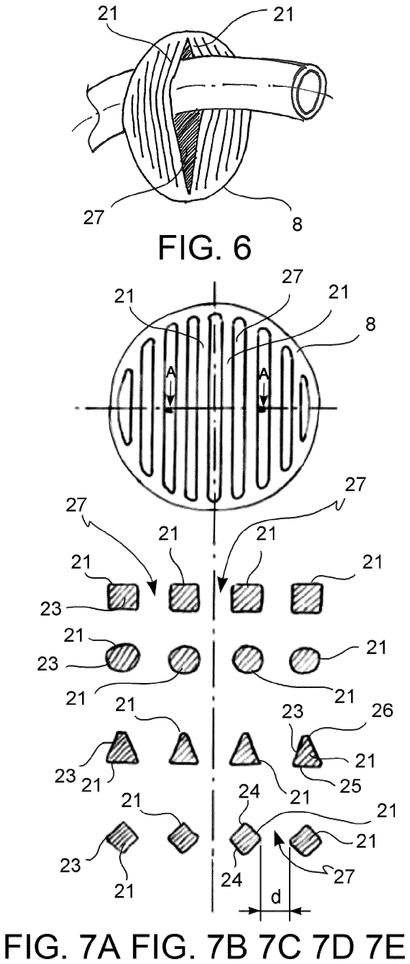

Depending on the technology adopted for the manufacture, the elastic fringes 21, and in particular the cross sections thereof, can be made in various shapes, as in the example in figures 7B to 7E, 8A and 8B, and 9.

The distance "d", shown in Figure 7E, between the fringes 21 can vary between zero (for example in diaphragms obtained from cut membranes) and a distance of the order of one millimeter. Distances of such an extent, indeed, are destined to be soon occluded by natural tissue regrowth.

The individual fringes 21 can have different shapes and orientation, and the distance therebetween may also be nonhomogeneous.

In terms of materials, thin-disc diaphragms 8 can typically be made of silicone or polyurethane, choosing the types the physical-mechanical features of which are most suitable for the intended use.

In technological terms, the diaphragms 8 with elastic fringes 21 can be obtained:
- from membranes then incised in the form of a fringe;
- by molding;
- by means of 3-D printing processes;
- by means of subsequent operations of gluing various components.

The diaphragm 8 in the shape of a thin disc can also be made from bioabsorbable polymers. In this case, the possibility of re-crossing will typically be obtained at the end of the bio-absorption process, due to the substantial replacement of the diaphragm 8 with the patient's natural tissue. By adopting particularly elastic bio-absorbable polymers, re-crossability will be ensured, immediately after implantation, by an operating mechanism which is similar to that of the silicone or polyurethane diaphragms (offset + elongation of fringes).

As regards the fixing of the diaphragm 8, for example in the center of the support structure 2, this can be done according to one of the techniques listed here:
- gluing
- heat sealing
- stitching
- co-molding
- crimping
- interlocking
- a mixed solution among the previous ones

1-b) diaphragm with occluding bridges in an "elastic fringe curtain" obtained with elastic threads.

A second embodiment for diaphragms 8 with elastic fringes 21 is that which can be obtained from elastic and highly extendable threads 40. Such a solution can be implemented ac-cording to various approaches. For example, in a first solution, the threads 40 may be applied directly to the structures of the support structure. Or, in a second example, the threads 40 may be applied to a frame, in turn mounted to the support structure.

The threads 40 may be applied so as to create different patterns: parallel, crossed, radial threads.

In terms of materials, the same materials listed for the thin diss solution, including bioerodible polymers, are suitable for these threads 40.

The threads 40 can be obtained by extrusion or other known techniques and applied to the frames or directly to the support structure 2 by:
- winding
- binding
- heat sealing
- gluing
- pinching
- other known tech-niques
- mixed solutions among those listed

1-c) diaphragm with occluding bridges in a "dome of elastic fringes", defining an embodiment not part of the present invention.

Physiologically, pressure differences exist between the two sides of the atrial partition, which vary within the cardiac cycle. In particular, the pressure in left atrium 102 is higher than that in the right atrium 101. A constructional solution aimed at minimizing the passage of blood from the chamber, or compartment, at a higher pressure to that at a lower pressure can be that of the "dome of elastic fringes", defining an embodiment not part of the present invention.

The solution essentially follows that of the disc of elastic fringes described in item 1-a but, instead of developing flat, it precisely develops in the shape of a dome, defining an embodiment not part of the present invention. The dome will be mounted to the support structure with the convexity facing the higher pressure chamber. Thereby, the blood itself, at a higher pressure, will tend to compact the adjacent fringes 21 together, thus increasing the tightness of the occlusion.

As for the materials, the technologies for making the dome, defining an embodiment not part of the present invention, and how to fix it to the frame, the same related to the disc solution described above applies.

Some exemplary embodiment of an occluding device 1 according to the present invention will be described below.

. In particular, the solution described herein has an "integral solution", or in a single piece, (support structure 2 + diaphragm 8) made of medical elastomer, for example silicone or polyurethane.

In general terms, the description of the device coincides with that given in the previous embodiments, taking into account that in this embodiment the support structure does not derive from known solutions made of metal alloy, but is made of medical elastomer.

The advantages of this solution compared to those already described mainly lie in the high simplicity and cost-effectiveness of the construction, as well as in a greater lightness compared to the traditional occluders obtained from metal alloys.

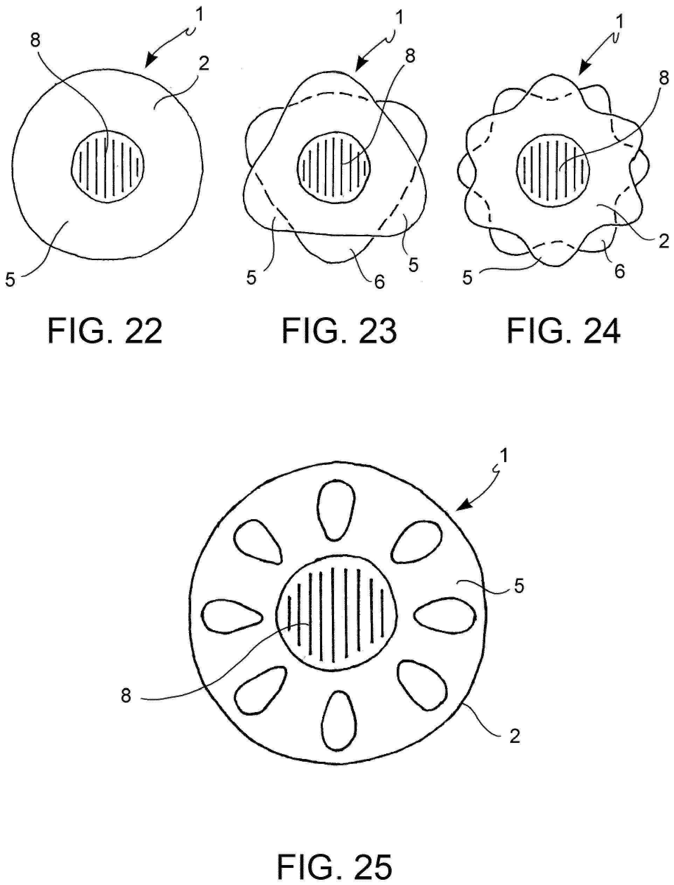

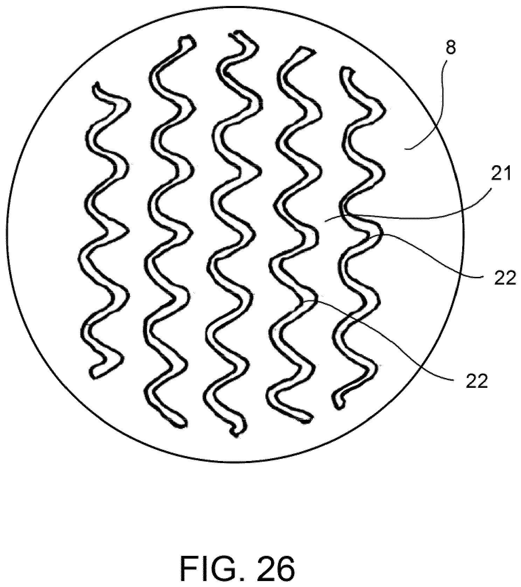

In Figures 22 to 30, the diaphragm 8 is depicted in its shape of a "curtain of elastic fringes", but it will be able to be made according to any of the solutions described above.

The general structure of the device reflects that already described, which includes:
- a support structure 2 dedicated to the positioning and anchoring in situ of the device 1, generally toroidal in shape with opposing "umbrellas" or "donuts", positioned astride the defect or hole, with the umbrellas or donuts arranged on opposite sides of the interatrial partition;
- a diaphragm 8 or central diaphragm dedicated to the occlusion of the iatrogenic or congenital defect/hole, with the possibility of being crossed again after some time.

In this case, the support structure 2 is made of medical elastomer, and is characterized by a specific design, adapted to utilize the features of that family of materials and to optimize the functionality of the device:
- compression along the ridge;
- convexity facing the atrial chamber.

The support structure 2, in accordance with an embodiment, has a convexity facing the atrial chambers. This peculiarity offers a range of advantages:
- a gap is created between the support structure and the interatrial partition. In this gap, the tissue of the atrial partition comes out during the expansion and in-situ release of the device. In fact, it must be considered that the iatrogenic or congenital defect or hole to be occluded will have a typically smaller diameter than that of the occluder, and an irregular shape. Under the radial thrust of the occluder, the excess tissue will widen and deform, and will be accommodated in the gap provided between the anchoring portions. In the absence of this gap, the excess tissue could hinder the regular expansion of the occluder device 1;
- due to the shape provided, the support structure 2 rests on the interatrial partition mainly along the external edge only, concentrating along such an edge the compression force which stabilizes the device 1 in place. Thereby, the contact pressure is high, and the adhesion of the support structure 2 to the partition is optimal.

The above effects can be modulated by acting on dimensions and other measures as shown in Figures 23 to 26.

In particular, the thickening of the edge (Figure 24), or the inclusion of a reinforcing material (Figure 25), can strongly increase the resistance to displacement.

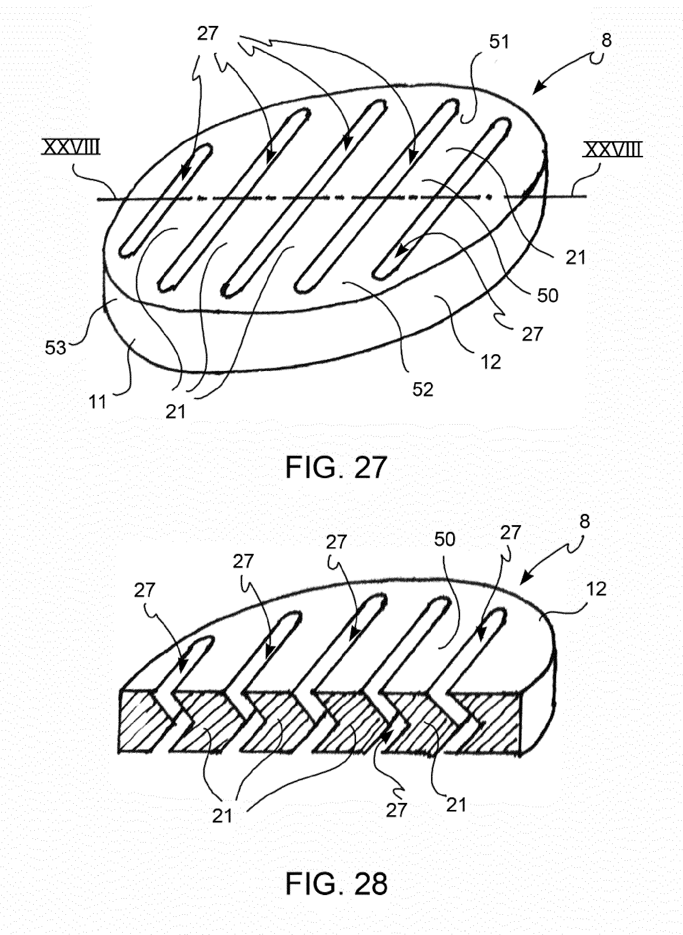

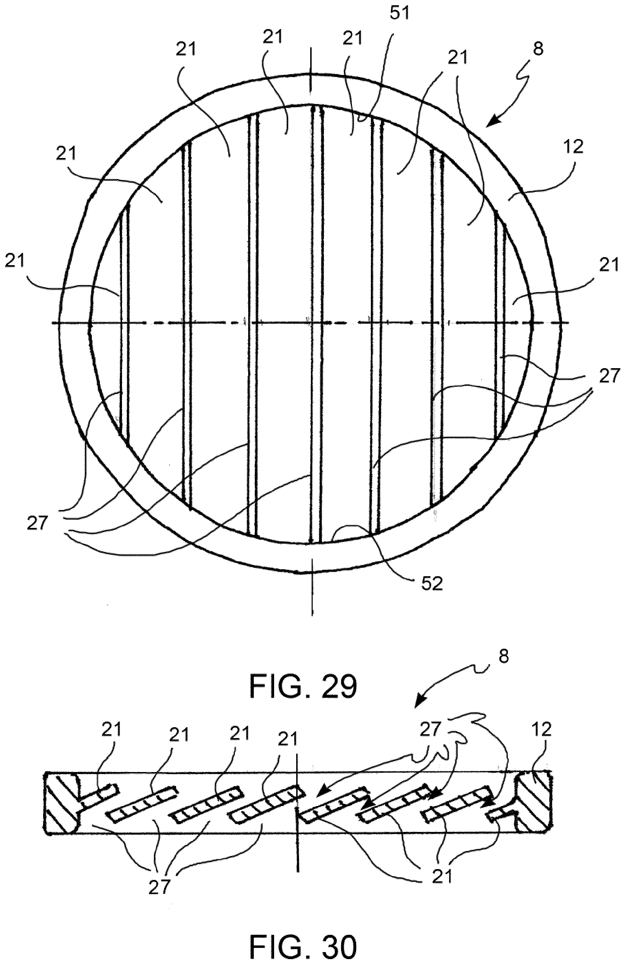

Even in terms of the general shape of the support structure, it is possible to manufacture the device according to various solutions, according to the desired performance. For example, lobed forms for the frame umbrellas, with lobes staggered between the two sides of the partition, can facilitate the anchoring and the ease of "crimping" the device on a delivery catheter (Figures 28 and 29).

It is also possible to vary the design of the umbrellas by making cuts or openings in appropriate shapes and positions. Thereby, the device 1 is lightened, the creation of massive thrombotic formations between the umbrella and the interatrial partition is avoided, and the "crimping" of the device on the delivery catheter is facilitated (Figure 30).

In terms of materials, the whole device 1 is made of medical elastomer, typically silicone or polyurethane. Alternatively, the whole device 1 is made of bio-erodible polymers.

The preferred solution is obtained by molding, according to known techniques, the component in a single piece.

In accordance with an embodiment, the device can also be obtained by assembling, typically by gluing, parts obtained with elastomers which are different in hardness or elasticity. For example, the support structure 2 could be made of a single piece of more rigid elastomer, by applying a more pliable elastomer diaphragm 8 in the center.

There is also the possibility of co-molding, inserting, applying other local reinforcement components, or intended for other functions, made of different materials: metallic alloys, polymer threads, fabrics, and the like.

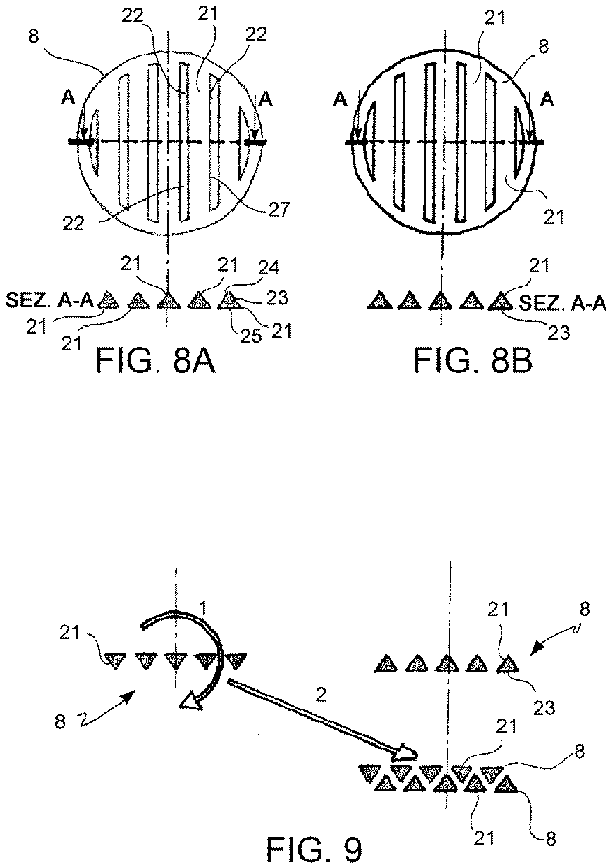

In accordance with an alternative embodiment, two fringed diaphragms 8 are provided, the fringes 21 of which have a tapered cross section, for example triangular or trapezoidal. Said two diaphragms 8 overlap with each other and are slightly staggered, so as to intercalate the respective fringes 21 and arrange the triangular or rhomboidal sections of either diaphragm at least partially interpenetrated to improve the fluid tightness, as shown in Figures 8A, 8B, 9, for example.

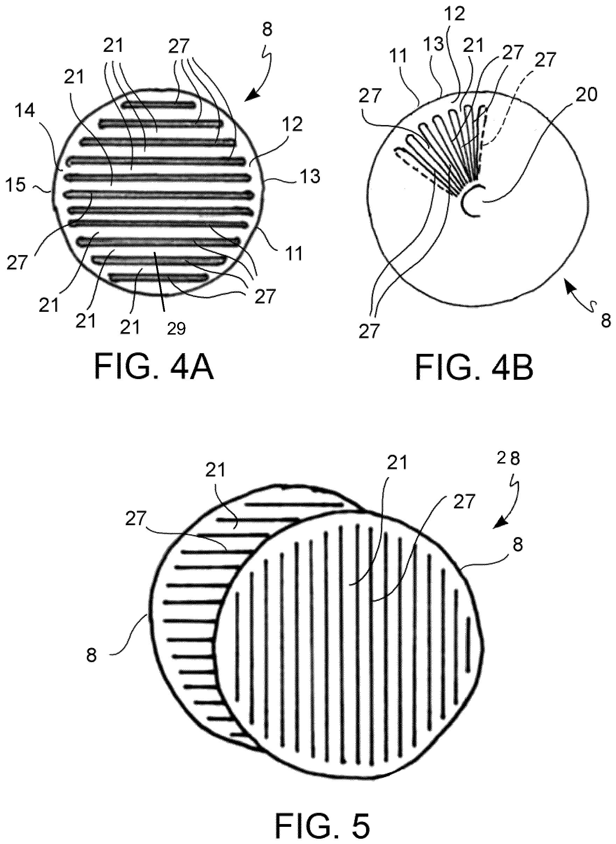

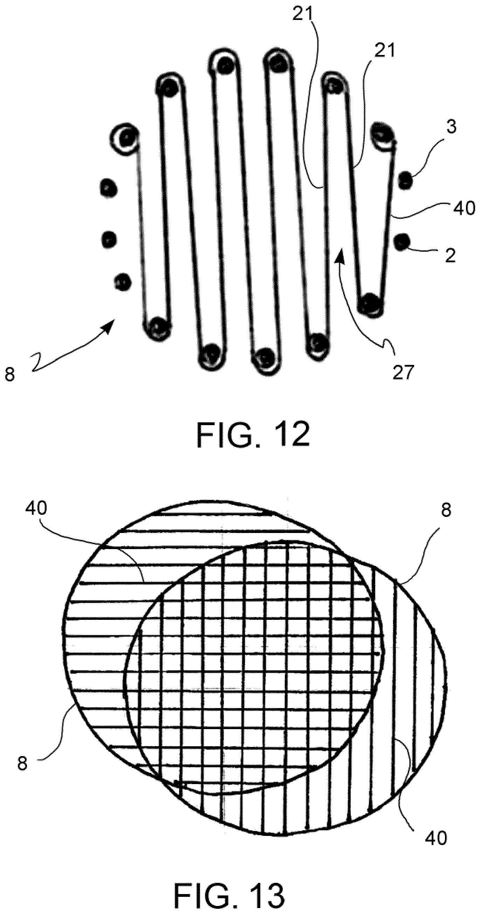

In accordance with an alternative embodiment, two fringed diaphragms 8 are provided. Said two fringed diaphragms 8 overlap with each other by angularly arranging the fringes 21 of a diaphragm 8 crossed with respect to the fringes 21 of the other diaphragm 8. For example, the fringes 21 of a first diaphragm 8 are arranged orthogonal to the fringes 21 of the second diaphragm 8, as shown in Figure 5 or in Figure 13, for example, where the fringes are in a thread.

In accordance with an alternative embodiment, a diaphragm 8 with fringes 21 in a thread is provided. Said thread-like fringes 21 are mounted to frame and said frame is fixed to said support structure 2.

In accordance with an alternative embodiment, a diaphragm 8 with fringes 21 having a wavy fringe body is provided, thus allowing each fringe urged by a possible crossing of the diaphragm, to elongate, and then return to a closed position of the diaphragm 8 itself.

In accordance with an alternative embodiment, a diaphragm 8 with fringes 21 having wavy fringe edges is provided, the fringe edge being the lateral portion of the fringe which delimits said elongated and narrow opening 27, thus allowing each fringe urged by a possible crossing of the diaphragm by a medical device, to elongate, but facilitating, once the device has been removed, the re-closing of the fringes 21 and therefore of the diaphragm 8.

All different embodiments of diaphragm 8 and fringes 21 thereof described above advantageously allow the re-crossing of the diaphragm 8, after its implantation, and equally advantageously, after the removal of said medical device, allow the re-closing of the fringes 21 and therefore the re-closing of the diaphragm 8 itself.

### LIST OF REFERENCE NUMERALS

- 1: crossable interseptal occluder device
- 2: support structure
- 3: central support structure portion
- 4: lumen
- 5: first anchoring portion
- 6: second anchoring portion
- 7: partition
- 8: diaphragm
- 9: first compartment
- 10: second compartment
- 11: diaphragm edge
- 12: first diaphragm portion or external continuous annular diaphragm portion
- 13: first edge stretch
- 14: second diaphragm portion
- 15: second edge stretch
- 16: annular path
- 17: small diaphragm bridges
- 18: small diaphragm bridges
- 19: small diaphragm bridges
- 20: central diaphragm portion
- 21: elongated membrane fringes or occluding bridges
- 22: membrane fringe edges or occluding bridge edges or occluding bridge sides
- 23: fringe section or bridge section
- 24: section end
- 25: first section base side
- 26: smaller section side
- 27: elongated and narrow opening or cut
- 28: pair of diaphragms
- 29: central fringe portion or central occluding bridge portion
- 30: diaphragm stiffening structure
- 31: diaphragm ribs
- 32: diametrical rib
- 33: circumferentially extending rib
- 34: radially extending rib
- 35: rib extending along a circle chord
- 36: diaphragm center
- 38: diaphragm half-dome
- 39: diaphragm dome
- 40: elastic threads or single elastic thread
- 41: elastic thread stretches
- 42: plurality of elastic threads
- 50: occluding bridge body
- 51: occluding bridge body end
- 52: occluding bridge body end
- 53: single sheet forming said diaphragm
- 54: first sheet edge
- 55: second sheet edge
- 100: atrial partition
- 101: right atrium
- 102: left atrium
- 103: heart
- 104: ventricular partition
- 105: right ventricle
- 106: left ventricle
- 107: defect or opening

## Claims

1. A crossable interseptal occluder device (1) comprising:
- a support structure (2); wherein
- said support structure (2) comprises a central support structure portion (3) which delimits a lumen (4);
- said support structure (2) comprises a first anchoring portion (5) and an opposite second anchoring portion (6);
- said support structure (2) being configured to expand and contract between a compressed tubular configuration for the insertion through the patient's vasculature, and an expanded configuration, in which the first and second anchoring portions (5, 6) extend radially towards the outside from said central support structure portion (3) to compress a partition (7) therebetween arranging said support structure (2) astride said partition (7) through a defect or hole or opening present in the partition (7);
and wherein
- said crossable interseptal occluder device (1) further comprises at least one diaphragm (8);
- said diaphragm (8) being supported by said support structure (2) and arranged to occlude the majority of said lumen (4) when said diaphragm (8) is in a relaxed configuration;
- said diaphragm (8) being configured to allow a medical instrument inserted into a first compartment (9 or 10) delimited by said partition (7) to pass through the diaphragm (8) and then through said lumen (4) entering a second compartment (10 or 9) delimited by said partition (7);
wherein
- said diaphragm (8) comprises a plurality of occluding bridges (21) ;
- each of said plurality of occluding bridges (21) comprises an elongated body (50) having opposite elongated body ends (51, 52), and opposite longitudinal edges (22);
- at least a portion of said longitudinal edges (22) of any occluding bridge (21) and at least a portion of said longitudinal edge (22) of a further occluding bridge (21) delimiting at least a slit or a cut (27) into said diaphragm (8);
wherein
- both said opposite ends (51, 52) of each occluding bridge (21) are directly or indirectly connected to or supported by said support structure (2);
wherein when under relaxed conditions, said diaphragm (8) is planar in shape and said occluding bridges (21) are tensioned on said support structure (2);
- in a relaxed configuration of said diaphragm (8), each occluding bridge (21) of said plurality of occluding bridges (21) is disposed close to at least said further occluding bridge (21) of said plurality of occluding bridges (21);
and wherein said diaphragm (8) is an elastic membrane; and wherein said occluding bridges (21) are configured to move away and extend to allow said medical instrument to pass through the diaphragm (8).

2. A crossable interseptal occluder device (1) according to claim 1, wherein
- in a relaxed configuration of said diaphragm (8), each of said opposite longitudinal edges (22) delimits, with the edge of an adjacent occluding bridge (21), an elongated and narrow opening or elongated slit (27).

3. A crossable interseptal occluder device (1) according to claim 1 or 2, wherein
- in a relaxed configuration of said diaphragm (8), each of said plurality of occluding bridges (21) of each diaphragm of said at least one diaphragm (8) rests in a single plane.

4. A crossable interseptal occluder device (1) according to anyone of the preceding claims, wherein
- in a relaxed configuration of said diaphragm (8), each occluding bridge (21) is disposed close to at least a further occluding bridge (21) facing with at least a portion of its longitudinal edge (22) at least a portion of a longitudinal edge (22) of said further occluding bridge (21).

5. A crossable interseptal occluder device (1) according to anyone of the preceding claims, wherein
in a relaxed configuration of said diaphragm (8), each of said opposite longitudinal edges (22) rests in a manner that the sum of the elongated body (50) of said occluding bridges (21) occludes the majority of said lumen (4).

6. A crossable interseptal occluder device (1) according to anyone of the preceding claims, wherein
in a relaxed configuration of said diaphragm (8), said slit or cut (27) has a longitudinal extension equal to the entire longitudinal extension of said elongated body (50) of each occluding bridge (21) delimiting said slit or cut (27).

7. A crossable interseptal occluder device (1) according to anyone of the preceding claims, wherein
- both of said opposite ends (51, 52) of each occluding bridge (21) is directly or indirectly connected to or supported by said central support structure portion (3) of said support structure (2).

8. A crossable interseptal occluder device (1) according to anyone of the preceding claims, wherein
said diaphragm (8) is made of elastic material capable of a deformation of at least in the range of 10% or 20%;
or
- said diaphragm (8) is made of silicone or medical elastomer or polyurethane or bio-erodible material; and wherein said diaphragm (8) is made of elastic material capable of a deformation of at least 150%.

9. A crossable interseptal occluder device (1) according to anyone of the preceding claims, wherein said occluding bridges (21) of said diaphragm (8) are separated from one another in the occluding bridge extension or central portion (29) of said occluding bridges (27) but joined together in a single piece.

10. A crossable interseptal occluder device (1) according to any one of the preceding claims, wherein
- at least one of said occluding bridges (21) has a longitudinal extension and comprises an occluding bridge section (23) evaluated in a direction transverse to said longitudinal direction of circular shape;
or wherein
- at least one of said occluding bridges (21) has a longitudinal extension and comprises an occluding bridge section (23) evaluated in a direction transverse to said longitudinal direction of square or rectangular shape;
or wherein
- at least one of said occluding bridges (21) has a longitudinal extension and comprises an occluding bridge section (23) evaluated in a direction transverse to said longitudinal direction of tapered shape passing from the center of said section to a section end (24) thereof;
or wherein
- at least one of said occluding bridges (21) has a longitudinal extension and comprises an occluding bridge section (23) evaluated in a direction transverse to said longitudinal direction of tapered shape passing from a first section base side (25) to a section end (24) ;
or wherein
- at least one of said occluding bridges (21) has a longitudinal extension and comprises an occluding bridge section (23) evaluated in a direction transverse to said longitudinal direction of tapered shape passing from a first section base side (25) to a smaller section side (26);
or wherein
- at least one of said occluding bridges (21) has a longitudinal extension and comprises an occluding bridge section (23) evaluated in a direction transverse to said longitudinal direction of oval or triangular or rhomboid or trapezoidal shape.

11. A crossable interseptal occluder device (1) according to any one of the preceding claims, wherein
- said device comprises a pair of diaphragms (28), each of said diaphragms (8) of said pair of diaphragms (28) comprising a plurality of occluding bridges (21);
and wherein
- said diaphragms (8) of said pair of diaphragms (28) are arranged facing each other and with the plurality of occluding bridges (21) of the first diaphragm (8) of said pair of diaphragms (28) offset with respect to the plurality of diaphragm occluding bridges (21) of the second diaphragm (8) of said pair of diaphragms (28) overlapping at least partially said plurality of occluding bridges (21) of the first diaphragm (8) to said plurality of elongated slits or cuts (27) of said second diaphragm (8).

12. A crossable interseptal occluder device (1) according to claim 11, wherein
- said plurality of occluding bridges (21) of said diaphragms (8) has tapered occluding bridge sections (23), for example triangular or rhomboid or arched;
and wherein
- the tapering of the occluding bridge sections (23) of said plurality of occluding bridges (21) of the first diaphragm (8) tapers towards said second diaphragm (8).

13. A crossable interseptal occluder device (1) according to claim 11 or 12, wherein
- said plurality of occluding bridges (21) of the first diaphragm (8) at least partially interpenetrates between said occluding bridges (21) of said plurality of occluding bridges (21) of the second diaphragm (8).

14. A crossable interseptal occluder device (1) according to any one of the preceding claims, wherein
- said diaphragm (8) comprises a diaphragm stiffening structure (30) ;
and wherein
- said diaphragm stiffening structure (30) comprises diaphragm ribs (31) adapted to create a supporting scaffold.

15. A crossable interseptal occluder device (1) according to claim 14, wherein
- said diaphragm stiffening structure (30) comprises at least one diametrical rib (32) extending along a diameter of said diaphragm (8) ;
or wherein
- said diaphragm stiffening structure (30) comprises at least one circumferentially extending rib (33);
or wherein
- said diaphragm stiffening structure (30) comprises at least one radially extending rib (34);
or wherein
- said diaphragm stiffening structure (30) comprises at least one rib extending along a circle chord (35).

16. A crossable interseptal occluder device (1) according to any one of the preceding claims, wherein
- said diaphragm (8) comprises a diaphragm external annular continuous portion (12) directly or indirectly connected to said supporting structure (2); and wherein
- each occluding bridge end (51, 52) is connected to said diaphragm external annular continuous portion (12);
or wherein
- said diaphragm (8) comprises a diaphragm external annular continuous portion (12) directly or indirectly connected to said supporting structure (2); and wherein
- each occluding bridge end (51, 52) is in one piece with said diaphragm external annular continuous portion (12).

17. A crossable interseptal occluder device (1) according to any one of claims 1 to 8 and 10 to 13 not depending from claim 9, wherein
- said diaphragm (8) comprises a plurality of elastic hollow tubes or wires or threads (42) comprising elastic hollow tube or wire or thread stretches (41) which form said diaphragm (8);
or wherein
- said diaphragm (8) comprises a single elastic hollow tube or wire or thread (40) folded to form elastic hollow tube or wire or thread stretches (41) which form said diaphragm (8).

18. A crossable interseptal occluder device (1) according to claim 17, wherein
- said diaphragm (8) comprises a plurality of elastic hollow tubes or wires or thread stretches (41) parallel to one another;
or wherein
said diaphragm (8) comprises a plurality of elastic hollow tubes or wires or thread stretches (41) mutually arranged side by side;
or wherein
- said diaphragm (8) comprises a plurality of elastic hollow tubes or wires or thread stretches (41) adjacent to one another;
or wherein
- said diaphragm (8) comprises a plurality of radially arranged elastic hollow tubes or wires or thread stretches (41);
and wherein
- said diaphragm (8) comprises a first plurality of elastic hollow tubes or wires or thread stretches (41) parallel to one another and a second plurality of elastic hollow tubes or wires or thread stretches (41) which in said second plurality are parallel to one another but orthogonal to said first plurality of elastic hollow tubes or wires or thread stretches (41);
or wherein
- said diaphragm (8) comprises a first plurality of elastic hollow tubes or wires or thread stretches (41) parallel to one another and at least a second plurality of elastic hollow tubes or wires or thread stretches (41) which in said at least a second plurality are parallel to one another but inclined with respect to said first plurality of elastic thread stretches (41).

19. A crossable interseptal occluder device (1) according to any one of claims 1 to 16, wherein
- said support structure (2) is in one piece;
- said support structure (2) and said diaphragm (8) are in one piece;
and/or wherein
- said support structure (2) is of medical elastomer.

20. A crossable interseptal occluder device (1) according to any one of the preceding claims, wherein
said device (1) comprises a diaphragm (8) with occluding bridges (21) having a wavy occluding bridge body (50);
or wherein
said device (1) comprises a diaphragm (8) with occluding bridges (21) having a wavy occluding bridge body (50), thus allowing each occluding bridge (21) urged by a possible crossing of the diaphragm itself, for example by a medical device, to elongate and then return to a closed position of the diaphragm (8);
or wherein
said device (1) comprises a diaphragm (8) with occluding bridges (21) having wavy occluding bridge edges (22), with occluding bridge edge meaning the side portion of the occluding bridge (21) which delimits said elongated slit (27), thus allowing each occluding bridge urged by a possible crossing of the diaphragm by a medical device, to elongate, and facilitating, once the device has been removed, the reclosure of the occluding bridges (21) and therefore of the diaphragm (8) itself.

21. A crossable interseptal occluder device (1) according to any one of claims 1 to 16 or 20, wherein said diaphragm (8) is obtained by a single foil wherein said elongated slits (27) are made by laser cuts.

## Patentansprüche

1. Durchquerbare interseptale Verschlussvorrichtung (1), umfassend:
- eine Halterungsstruktur (2); wobei
- die Halterungsstruktur (2) einen zentralen Halterungsstrukturabschnitt (3) umfasst, welcher ein Lumen (4) begrenzt;
- die Halterungsstruktur (2) einen ersten Verankerungsabschnitt (5) und einen entgegengesetzten zweiten Verankerungsabschnitt (6) umfasst;
- die Halterungsstruktur (2) dazu eingerichtet ist, sich auszudehnen und zusammenzuziehen zwischen einer komprimierten rohrförmigen Konfiguration für das Einführen durch das Gefäßsystem des Patienten und einer ausgedehnten Konfiguration, in welcher sich der erste und der zweite Verankerungsabschnitt (5, 6) von dem zentralen Halterungsstrukturabschnitt (3) radial in Richtung des Äußeren erstrecken, um eine Trennwand (7) dazwischen zu komprimieren, indem die Halterungsstruktur (2) durch eine Fehlstelle oder ein Loch oder eine Öffnung, welche in der Trennwand (7) vorhanden sind, rittlings der Trennwand (7) angeordnet wird,
und wobei
- die durchquerbare interseptale Verschlussvorrichtung (1) ferner wenigstens eine Membran (8) umfasst;
- die Membran (8) durch die Halterungsstruktur (2) gehaltert ist und derart angeordnet ist, dass sie den Großteil des Lumens (4) verschließt, wenn die Membran (8) in einer entspannten Konfiguration ist;
- die Membran (8) dazu eingerichtet ist, zu erlauben, dass ein medizinisches Instrument, welches in eine erste Kammer (9 oder 10) eingeführt wird, welche durch die Trennwand (7) begrenzt ist, die Membran (8) und dann das Lumen (4) zu durchdringen und in eine zweite Kammer (10 oder 9) einzudringen, welche durch die Trennwand (7) begrenzt ist;
wobei
- die Membran (8) eine Mehrzahl verschließender Brücken (21) umfasst;
- jede der Mehrzahl verschließender Brücken (21) einen länglichen Körper (50) umfasst, welcher entgegengesetzte längliche Körperenden (51, 52) und entgegengesetzte longitudinale Ränder (22) aufweist;
- wenigstens ein Abschnitt der longitudinalen Ränder (22) jeglicher verschließenden Brücke (21) und wenigstens ein Abschnitt des longitudinalen Rands (22) einer weiteren verschließenden Brücke (21) wenigstens einen Schlitz oder einen Einschnitt (27) in die Membran (8) begrenzen;
wobei
- die beiden entgegengesetzten Enden (51, 52) jeder verschließenden Brücke (21) direkt oder indirekt mit der Halterungsstruktur (2) verbunden sind oder dadurch gehaltert sind;
wobei unter entspannten Bedingungen die Membran (8) in ihrer Form planar ist und die verschließenden Brücken (21) an jeder Halterungsstruktur (2) gespannt sind;
- in einer entspannten Konfiguration der Membran (8) jede verschließende Brücke (21) der Mehrzahl verschließender Brücken (21) nahe wenigstens der weiteren verschließenden Brücke (21) der Mehrzahl verschließender Brücken (21) angeordnet ist;
und wobei die Membran (8) eine elastische Membran ist; und wobei die verschließenden Brücken (21) dazu eingerichtet sind, sich wegzubewegen und sich derart zu erstrecken, dass sie erlauben, dass das medizinische Instrument die Membran (8) durchdringt.

2. Durchquerbare interseptale Verschlussvorrichtung (1) nach Anspruch 1, wobei
- in einer entspannten Konfiguration der Membran (8) jeder der entgegengesetzten longitudinalen Ränder (22) mit dem Rand einer benachbarten verschließenden Brücke (21) eine längliche und schmale Öffnung oder einen länglichen Schlitz (27) begrenzt.

3. Durchquerbare interseptale Verschlussvorrichtung (1) nach Anspruch 1 oder 2, wobei
- in einer entspannten Konfiguration der Membran (8) jede der Mehrzahl verschließender Brücken (21) jeder Membran der wenigstens einen Membran (8) in einer einzigen Ebene liegt.

4. Durchquerbare interseptale Verschlussvorrichtung (1) nach einem der vorhergehenden Ansprüche, wobei
- in einer entspannten Konfiguration der Membran (8) jede verschließende Brücke (21) nahe wenigstens einer weiteren verschließenden Brücke (21) angeordnet ist, welche mit wenigstens einem Abschnitt ihres longitudinalen Rands (22) wenigstens einem Abschnitt eines longitudinalen Rands (22) der weiteren verschließenden Brücke (21) zugewandt ist.

5. Durchquerbare interseptale Verschlussvorrichtung (1) nach einem der vorhergehenden Ansprüche, wobei
in einer entspannten Konfiguration der Membran (8) jeder der entgegengesetzten longitudinalen Ränder (22) in einer Weise liegt, dass die Summe des länglichen Körpers (50) der verschließenden Brücken (21) den Großteil des Lumens (4) verschließt.

6. Durchquerbare interseptale Verschlussvorrichtung (1) nach einem der vorhergehenden Ansprüche, wobei
in einer entspannten Konfiguration der Membran (8) der Schlitz oder Einschnitt (27) eine longitudinale Erstreckung aufweist, welche gleich der gesamten longitudinalen Erstreckung des länglichen Körpers (50) jeder verschließenden Brücke (21) ist, welche den Schlitz oder Einschnitt (27) begrenzt.

7. Durchquerbare interseptale Verschlussvorrichtung (1) nach einem der vorhergehenden Ansprüche, wobei
- beide der entgegengesetzten Enden (51, 52) jeder verschließenden Brücke (21) direkt oder indirekt mit dem zentralen Halterungsstrukturabschnitt (3) der Halterungsstruktur (2) verbunden sind oder dadurch gehaltert sind.

8. Durchquerbare interseptale Verschlussvorrichtung (1) nach einem der vorhergehenden Ansprüche, wobei
die Membran (8) aus einem elastischen Material hergestellt ist, welches zu einer Verformung von wenigstens in dem Bereich von 10% oder 20% in der Lage ist;
oder
- die Membran (8) aus Silikon oder einem medizinischen Elastomer oder Polyurethan oder einem bio-erodierbaren Material hergestellt ist; und wobei die Membran (8) aus einem elastischen Material hergestellt ist, welches zu einer Verformung von wenigstens 150% in der Lage ist.

9. Durchquerbare interseptale Verschlussvorrichtung (1) nach einem der vorhergehenden Ansprüche, wobei die verschließenden Brücken (21) der Membran (8) in der Erstreckung der verschließenden Brücke oder dem zentralen Abschnitt (29) der verschließenden Brücken (27) voneinander getrennt sind, aber in einem einzigen Stück zusammengefügt sind.

10. Durchquerbare interseptale Verschlussvorrichtung (1) nach einem der vorhergehenden Ansprüche, wobei
- wenigstens eine der verschließenden Brücken (21) eine longitudinale Erstreckung aufweist und einen in einer zu der longitudinalen Richtung transversalen Richtung evaluierten Verschließende-Brücke-Abschnitt (23) von einer kreisförmigen Form umfasst;
oder wobei
- wenigstens eine der verschließenden Brücken (21) eine longitudinale Erstreckung aufweist und einen in einer zu der longitudinalen Richtung transversalen Richtung evaluierten Verschließende-Brücke-Abschnitt (23) von einer quadratischen oder einer rechteckigen Form umfasst;
oder wobei
- wenigstens eine der verschließenden Brücken (21) eine longitudinale Erstreckung aufweist und einen in einer zu der longitudinalen Richtung transversalen Richtung evaluierten Verschließende-Brücke-Abschnitt (23) von einer verjüngten Form umfasst, welche von dem Zentrum des Abschnitts zu einem Abschnittsende (24) davon verläuft;
oder wobei
- wenigstens eine der verschließenden Brücken (21) eine longitudinale Erstreckung aufweist und einen in einer zu der longitudinalen Richtung transversalen Richtung evaluierten Verschließende-Brücke-Abschnitt (23) von einer verjüngten Form umfasst, welche von einer ersten Abschnittsbasisseite (25) zu einem Abschnittsende (24) verläuft;
oder wobei
- wenigstens eine der verschließenden Brücken (21) eine longitudinale Erstreckung aufweist und einen in einer zu der longitudinalen Richtung transversalen Richtung evaluierten Verschließende-Brücke-Abschnitt (23) von einer verjüngten Form umfasst, welche von einer ersten Abschnittsbasisseite (25) zu einer kleineren Abschnittsseite (26) verläuft;
oder wobei
- wenigstens eine der verschließenden Brücken (21) eine longitudinale Erstreckung aufweist und einen in einer zu der longitudinalen Richtung transversalen Richtung evaluierten Verschließende-Brücke-Abschnitt (23) von einer ovalen oder einer dreieckigen oder einer rautenförmigen oder einer trapezförmigen Form umfasst.

11. Durchquerbare interseptale Verschlussvorrichtung (1) nach einem der vorhergehenden Ansprüche, wobei
- die Vorrichtung ein Paar von Membranen (28) umfasst, wobei jede der Membranen (8) des Paars von Membranen (28) eine Mehrzahl verschließender Brücken (21) umfasst;
und wobei
- die Membranen (8) des Paars von Membranen (28) einander zugewandt und mit der Mehrzahl verschließender Brücken (21) der ersten Membran (8) des Paars von Membranen (28), welche in Bezug auf die Mehrzahl Membranverschließender Brücken (21) der zweiten Membran (8) des Paars von Membranen (28) versetzt sind, wenigstens teilweise die Mehrzahl verschließender Brücken (21) der ersten Membran (8) zu der Mehrzahl länglicher Schlitze oder Einschnitte (27) der zweiten Membran (8) überlappend angeordnet sind.

12. Durchquerbare interseptale Verschlussvorrichtung (1) nach Anspruch 11, wobei
- die Mehrzahl verschließender Brücken (21) der Membranen (8) verjüngte Verschließende-Brücke-Abschnitte (23), zum Beispiel dreieckig oder rautenförmig oder gewölbt, aufweist;
und wobei
- die Verjüngung der Verschließende-Brücke-Abschnitte (23) der Mehrzahl verschließender Brücken (21) der ersten Membran (8) in Richtung der zweiten Membran (8) verjüngt ist.

13. Durchquerbare interseptale Verschlussvorrichtung (1) nach Anspruch 11 oder 12, wobei
- die Mehrzahl verschließender Brücken (21) der ersten Membran (8) wenigstens teilweise zwischen die verschließenden Brücken (21) der Mehrzahl verschließender Brücken (21) der zweiten Membran (8) durchdringt.

14. Durchquerbare interseptale Verschlussvorrichtung (1) nach einem der vorhergehenden Ansprüche, wobei
- die Membran (8) eine Membranversteifungsstruktur (30) umfasst;
und wobei
- die Membranversteifungsstruktur (30) Membranrippen (31) umfasst, welche dazu eingerichtet sind, ein Halterungsgerüst zu erzeugen.

15. Durchquerbare interseptale Verschlussvorrichtung (1) nach Anspruch 14, wobei
- die Membranversteifungsstruktur (30) wenigstens eine diametrale Rippe (32) umfasst, welche sich entlang eines Durchmessers der Membran (8) erstreckt; oder wobei
- die Membranversteifungsstruktur (30) wenigstens eine sich in Umfangsrichtung erstreckende Rippe (33) umfasst;
oder wobei
- die Membranversteifungsstruktur (30) wenigstens eine sich radial erstreckende Rippe (34) umfasst;
oder wobei
- die Membranversteifungsstruktur (30) wenigstens eine Rippe umfasst, welche sich entlang eines Kreisakkords (35) erstreckt.

16. Durchquerbare interseptale Verschlussvorrichtung (1) nach einem der vorhergehenden Ansprüche, wobei
- die Membran (8) einen äußeren ringförmigen kontinuierlichen Membranabschnitt (12) umfasst, welcher direkt oder indirekt mit der Halterungsstruktur (2) verbunden ist; und wobei
- jedes Verschließende-Brücke-Ende (51, 52) mit dem äußeren ringförmigen kontinuierlichen Membranabschnitt (12) verbunden ist;
oder wobei
- die Membran (8) einen äußeren ringförmigen kontinuierlichen Membranabschnitt (12) umfasst, welcher direkt oder indirekt mit der Halterungsstruktur (2) verbunden ist; und wobei
- jedes Verschließende-Brücke-Ende (51, 52) in einem Stück mit dem äußeren ringförmigen kontinuierlichen Membranabschnitt (12) vorliegt.

17. Durchquerbare interseptale Verschlussvorrichtung (1) nach einem der Ansprüche 1 bis 8 und 10 bis 13, welcher nicht abhängig von Anspruch 9 ist, wobei
- die Membran (8) eine Mehrzahl elastischer hohler Rohre oder Drähte oder Fäden (42) umfasst, die elastische hohle Rohr- oder Draht- oder Fadenabschnitte (41) umfassen, welche die Membran (8) bilden;
oder wobei
- die Membran (8) ein einziges elastisches hohles Rohr (40) oder einen einzigen elastischen hohlen Draht (40) oder einen einzigen elastischen hohlen Faden (40) umfasst, welcher gefaltet ist, um elastische hohle Rohr- oder Draht- oder Fadenabschnitte (41) zu bilden, welche die Membran (8) bilden.

18. Durchquerbare interseptale Verschlussvorrichtung (1) nach Anspruch 17, wobei
- die Membran (8) eine Mehrzahl elastischer hohler Rohr- oder Draht- oder Fadenabschnitte (41) umfasst, welche parallel zueinander sind;
oder wobei
die Membran (8) eine Mehrzahl elastischer hohler Rohr- oder Draht- oder Fadenabschnitte (41) umfasst, welche nebeneinander angeordnet sind;
oder wobei
- die Membran (8) eine Mehrzahl elastischer hohler Rohr- oder Draht- oder Fadenabschnitte (41) umfasst, welche einander benachbart sind;
oder wobei
- die Membran (8) eine Mehrzahl radial angeordneter elastischer hohler Rohr- oder Draht- oder Fadenabschnitte (41) umfasst;
und wobei
- die Membran (8) eine erste Mehrzahl elastischer hohler Rohr- oder Draht- oder Fadenabschnitte (41), welche parallel zueinander sind, und eine zweite Mehrzahl elastischer hohler Rohr- oder Draht- oder Fadenabschnitte (41) umfasst, welche in der zweiten Mehrzahl parallel zueinander aber orthogonal zu der ersten Mehrzahl elastischer hohler Rohr- oder Draht- oder Fadenabschnitte (41) sind;
oder wobei
- die Membran (8) eine erste Mehrzahl elastischer hohler Rohr- oder Draht- oder Fadenabschnitte (41), welche parallel zueinander sind, und wenigstens eine zweite Mehrzahl elastischer hohler Rohr- oder Draht- oder Fadenabschnitte (41) umfasst, welche in der wenigstens einen zweiten Mehrzahl parallel zueinander aber geneigt in Bezug auf die erste Mehrzahl elastischer Fadenabschnitte (41) sind.

19. Durchquerbare interseptale Verschlussvorrichtung (1) nach einem der Ansprüche 1 bis 16, wobei
- die Halterungsstruktur (2) in einem Stück vorliegt;
- die Halterungsstruktur (2) und die Membran (8) in einem Stück vorliegen; und/oder wobei
- die Halterungsstruktur (2) aus einem medizinischen Elastomer ist.

20. Durchquerbare interseptale Verschlussvorrichtung (1) nach einem der vorhergehenden Ansprüche, wobei
die Vorrichtung (1) eine Membran (8) mit verschließenden Brücken (21) umfasst, welche einen welligen Verschließende-Brücke-Körper (50) aufweisen;
oder wobei
die Vorrichtung (1) eine Membran (8) mit verschließenden Brücken (21) umfasst, welche einen welligen Verschließende-Brücke-Körper (50) aufweisen, wodurch erlaubt wird, dass sich jede verschließende Brücke (21), welche durch ein mögliches Durchqueren der Membran selbst, zum Beispiel durch eine medizinische Vorrichtung, gedrückt wird, verlängert und dann zu einer geschlossenen Position der Membran (8) zurückkehrt;
oder wobei
die Vorrichtung (1) eine Membran (8) mit verschließenden Brücken (21) umfasst, welche wellige Verschließende-Brücke-Ränder (22) aufweisen, wobei mit Verschließende-Brücke-Rand der seitliche Abschnitt der verschließenden Brücke (21) gemeint ist, welcher den länglichen Schlitz (27) begrenzt, wodurch erlaubt wird, dass sich jede verschließende Brücke verlängert, welche durch ein mögliches Durchqueren der Membran durch eine medizinische Vorrichtung gedrückt wird, und erleichtert wird, dass sich, sobald sich die Vorrichtung zurückbewegt hat, die verschließenden Brücken (21) und daher die Membran (8) selbst wieder schließen.

21. Durchquerbare interseptale Verschlussvorrichtung (1) nach einem der Ansprüche 1 bis 16 oder 20, wobei die Membran (8) durch eine einzige Folie erhalten wird, wobei die länglichen Schlitze (27) durch Laserschnitte hergestellt werden.

## Revendications

1. Dispositif d'occlusion interseptale pouvant être traversé (1), comprenant :
- une structure de support (2) ; dans lequel
- ladite structure de support (2) comprend une portion de structure de support centrale (3) qui délimite une lumière (4) ;
- ladite structure de support (2) comprend une première portion d'ancrage (5) et une deuxième portion d'ancrage (6) opposée ;
- ladite structure de support (2) étant configurée pour se déployer et se contracter entre une configuration tubulaire comprimée pour l'insertion à travers le système vasculaire du patient, et une configuration déployée, dans laquelle les première et deuxième portions d'ancrage (5, 6) s'étendent radialement vers l'extérieur à partir de ladite portion de structure de support centrale (3) pour comprimer une cloison (7) entre les deux, agençant ladite structure de support (2) à cheval sur ladite cloison (7) via un défaut ou un trou ou une ouverture présents dans la cloison (7) ;
et dans lequel
- ledit dispositif d'occlusion interseptale pouvant être traversé (1) comprend en outre au moins un diaphragme (8) ;
- ledit diaphragme (8) étant supporté par ladite structure de support (2) et agencé pour occlure la majeure partie de ladite lumière (4) lorsque ledit diaphragme (8) se trouve dans une configuration détendue ;
- ledit diaphragme (8) étant configuré pour permettre à un instrument médical inséré dans un premier compartiment (9 ou 10) délimité par ladite cloison (7) de passer à travers le diaphragme (8) et ensuite à travers ladite lumière (4), entrant dans un deuxième compartiment (10 ou 9) délimité par ladite cloison (7) ;
dans lequel
- ledit diaphragme (8) comprend une pluralité de ponts d'occlusion (21) ;
- chacun de ladite pluralité de ponts d'occlusion (21) comprend un corps allongé (50) présentant des extrémités de corps allongé opposées (51, 52), et des bords longitudinaux opposés (22) ;
- au moins une portion desdits bords longitudinaux (22) de tout pont d'occlusion (21) et au moins une portion dudit bord longitudinal (22) d'un autre pont d'occlusion (21) délimitant au moins une fente ou une entaille (27) dans ledit diaphragme (8) ;
dans lequel
- lesdites extrémités opposées (51, 52) de chaque pont d'occlusion (21) sont toutes deux directement ou indirectement reliées à ladite structure de support (2) ou supportées par celle-ci ;
dans lequel, dans des conditions détendues, ledit diaphragme (8) est de forme plane et lesdits ponts d'occlusion (21) sont tendus sur ladite structure de support (2) ;
- dans une configuration détendue dudit diaphragme (8), chaque pont d'occlusion (21) de ladite pluralité de ponts d'occlusion (21) est disposé à proximité d'au moins ledit autre pont d'occlusion (21) de ladite pluralité de ponts d'occlusion (21) ;
et dans lequel ledit diaphragme (8) est une membrane élastique ; et dans lequel lesdits ponts d'occlusion (21) sont configurés pour s'écarter et s'étendre afin de permettre audit instrument médical de passer à travers le diaphragme (8).

2. Dispositif d'occlusion interseptale pouvant être traversé (1) selon la revendication 1, dans lequel
- dans une configuration détendue dudit diaphragme (8), chacun desdits bords longitudinaux opposés (22) délimite, avec le bord d'un pont d'occlusion (21) adjacent, une ouverture allongée et étroite ou une fente allongée (27).

3. Dispositif d'occlusion interseptale pouvant être traversé (1) selon la revendication 1 ou 2, dans lequel
- dans une configuration détendue dudit diaphragme (8), chacun de ladite pluralité de ponts d'occlusion (21) de chaque diaphragme dudit diaphragme (8), au moins au nombre de un, repose dans un seul plan.

4. Dispositif d'occlusion interseptale pouvant être traversé (1) selon l'une quelconque des revendications précédentes, dans lequel
- dans une configuration détendue dudit diaphragme (8), chaque pont d'occlusion (21) est disposé à proximité d'au moins un autre pont d'occlusion (21) faisant face par au moins une portion de son bord longitudinal (22) à au moins une portion d'un bord longitudinal (22) dudit autre pont d'occlusion (21).

5. Dispositif d'occlusion interseptale pouvant être traversé (1) selon l'une quelconque des revendications précédentes, dans lequel
dans une configuration détendue dudit diaphragme (8), chacun desdits bords longitudinaux opposés (22) repose de façon à ce que la somme du corps allongé (50) desdits ponts d'occlusion (21) occlue la majeure partie de ladite lumière (4).

6. Dispositif d'occlusion interseptale pouvant être traversé (1) selon l'une quelconque des revendications précédentes, dans lequel
dans une configuration détendue dudit diaphragme (8), ladite fente ou entaille (27) présente une extension longitudinale égale à l'ensemble de l'extension longitudinale dudit corps allongé (50) de chaque pont d'occlusion (21) délimitant ladite fente ou entaille (27).

7. Dispositif d'occlusion interseptale pouvant être traversé (1) selon l'une quelconque des revendications précédentes, dans lequel
- lesdites extrémités opposées (51, 52) de chaque pont d'occlusion (21) sont toutes deux directement ou indirectement reliées à ladite portion de structure de support centrale (3) de ladite structure de support (2) ou supportées par celle-ci.

8. Dispositif d'occlusion interseptale pouvant être traversé (1) selon l'une quelconque des revendications précédentes, dans lequel
ledit diaphragme (8) est fait en un matériau élastique capable d'une déformation au moins dans la plage de 10 % ou 20 % ;
ou
- ledit diaphragme (8) est fait en silicone ou en élastomère médical ou en polyuréthane ou en un matériau bioérodable ; et dans lequel ledit diaphragme (8) est fait en un matériau élastique capable d'une déformation d'au moins 150 %.

9. Dispositif d'occlusion interseptale pouvant être traversé (1) selon l'une quelconque des revendications précédentes, dans lequel lesdits ponts d'occlusion (21) dudit diaphragme (8) sont séparés les uns des autres dans l'extension de pont d'occlusion ou une portion centrale (29) desdits ponts d'occlusion (27), mais réunis en un élément unique.

10. Dispositif d'occlusion interseptale pouvant être traversé (1) selon l'une quelconque des revendications précédentes, dans lequel
- au moins un desdits ponts d'occlusion (21) présente une extension longitudinale et comprend une section de pont d'occlusion (23) évaluée dans une direction transversale à ladite direction longitudinale de forme circulaire ;
ou dans lequel
- au moins un desdits ponts d'occlusion (21) présente une extension longitudinale et comprend une section de pont d'occlusion (23) évaluée dans une direction transversale à ladite direction longitudinale de forme carrée ou rectangulaire ;
ou dans lequel
- au moins un desdits ponts d'occlusion (21) présente une extension longitudinale et comprend une section de pont d'occlusion (23) évaluée dans une direction transversale à ladite direction longitudinale de forme conique en passant du centre de ladite section à une extrémité de section (24) de celle-ci ;
ou dans lequel
- au moins un desdits ponts d'occlusion (21) présente une extension longitudinale et comprend une section de pont d'occlusion (23) évaluée dans une direction transversale à ladite direction longitudinale de forme conique en passant d'un premier côté de base de section (25) à une extrémité de section (24) ;
ou dans lequel
- au moins un desdits ponts d'occlusion (21) présente une extension longitudinale et comprend une section de pont d'occlusion (23) évaluée dans une direction transversale à ladite direction longitudinale de forme conique passant d'un premier côté de base de section (25) à un plus petit côté de section (26) ;
ou dans lequel
- au moins un desdits ponts d'occlusion (21) présente une extension longitudinale et comprend une section de pont d'occlusion (23) évaluée dans une direction transversale à ladite direction longitudinale de forme ovale ou triangulaire ou rhombique ou trapézoïdale.

11. Dispositif d'occlusion interseptale pouvant être traversé (1) selon l'une quelconque des revendications précédentes, dans lequel
- ledit dispositif comprend une paire de diaphragmes (28), chacun desdits diaphragmes (8) de ladite paire de diaphragmes (28) comprenant une pluralité de ponts d'occlusion (21) ;
et dans lequel
- lesdits diaphragmes (8) de ladite paire de diaphragmes (28) sont agencés face à face, la pluralité de ponts d'occlusion (21) du premier diaphragme (8) de ladite paire de diaphragmes (28) étant décalée par rapport à la pluralité de ponts d'occlusion (21) de diaphragme du deuxième diaphragme (8) de ladite paire de diaphragmes (28) en chevauchant au moins partiellement ladite pluralité de ponts d'occlusion (21) du premier diaphragme (8) sur ladite pluralité de fentes ou d'entailles allongées (27) dudit deuxième diaphragme (8).

12. Dispositif d'occlusion interseptale pouvant être traversé (1) selon la revendication 11, dans lequel
- ladite pluralité de ponts d'occlusion (21) desdits diaphragmes (8) a des sections de pont d'occlusion (23) effilées, par exemple triangulaires ou rhomboïdes ou en arc ;
et dans lequel
- l'effilement des sections de pont d'occlusion (23) de ladite pluralité de ponts d'occlusion (21) du premier diaphragme (8) s'effile vers ledit deuxième diaphragme (8).

13. Dispositif d'occlusion interseptale pouvant être traversé (1) selon la revendication 11 ou 12, dans lequel
- ladite pluralité de ponts d'occlusion (21) du premier diaphragme (8) s'interpénètre au moins partiellement entre lesdits ponts d'occlusion (21) de ladite pluralité de ponts d'occlusion (21) du deuxième diaphragme (8).

14. Dispositif d'occlusion interseptale pouvant être traversé (1) selon l'une quelconque des revendications précédentes, dans lequel
- ledit diaphragme (8) comprend une structure de rigidification de diaphragme (30) ;
et dans lequel
- ladite structure de rigidification de diaphragme (30) comprend des nervures de diaphragme (31) adaptées pour créer un échafaudage de support.

15. Dispositif d'occlusion interseptale pouvant être traversé (1) selon la revendication 14, dans lequel
- ladite structure de rigidification de diaphragme (30) comprend au moins une nervure diamétrale (32) s'étendant suivant un diamètre dudit diaphragme (8) ;
ou dans lequel
- ladite structure de rigidification de diaphragme (30) comprend au moins une nervure (33) s'étendant circonférenciellement ;
ou dans lequel
- ladite structure de rigidification de diaphragme (30) comprend au moins une nervure (34) s'étendant radialement ;
ou dans lequel
- ladite structure de rigidification de diaphragme (30) comprend au moins une nervure s'étendant suivant une corde de cercle (35).

16. Dispositif d'occlusion interseptale pouvant être traversé (1) selon l'une quelconque des revendications précédentes, dans lequel
- ledit diaphragme (8) comprend une portion continue annulaire externe de diaphragme (12) reliée directement ou indirectement à ladite structure de support (2) ; et dans lequel
- chaque extrémité de pont d'occlusion (51, 52) est reliée à ladite portion continue annulaire externe de diaphragme (12) ;
ou dans lequel
- ledit diaphragme (8) comprend une portion continue annulaire externe de diaphragme (12) directement ou indirectement reliée à ladite structure de support (2) ; et dans lequel
- chaque extrémité de pont d'occlusion (51, 52) est d'un seul tenant avec ladite portion continue annulaire externe de diaphragme (12).

17. Dispositif d'occlusion interseptale pouvant être traversé (1) selon l'une quelconque des revendications 1 à 8 et 10 à 13 ne dépendant pas de la revendication 9,
dans lequel
- ledit diaphragme (8) comprend une pluralité de tubes creux ou fils ou fils élastiques (42) comprenant des étirements de tube creux ou fil ou fil élastique (41) qui forment ledit diaphragme (8) ;
ou dans lequel
- ledit diaphragme (8) comprend un seul tube creux ou fil ou fil élastique (40) plié pour former des étirements (41) de tube creux ou fil ou fil élastique qui forment ledit diaphragme (8).

18. Dispositif d'occlusion interseptale pouvant être traversé (1) selon la revendication 17, dans lequel
- ledit diaphragme (8) comprend une pluralité d'étirements de tube creux ou fil ou fil élastique (41) parallèles les uns aux autres ;
ou dans lequel
- ledit diaphragme (8) comprend une pluralité d'étirements de tube creux ou fil ou fil élastique (41) agencés mutuellement côte à côte ;
ou dans lequel
- ledit diaphragme (8) comprend une pluralité d'étirements de tube creux ou fil ou fil élastique (41) adjacents les uns aux autres ;
ou dans lequel
- ledit diaphragme (8) comprend une pluralité d'étirements de tube creux ou fil ou fil élastique (41) agencés radialement ;
et dans lequel
- ledit diaphragme (8) comprend une première pluralité d'étirements de tube creux ou fil ou fil élastique (41) parallèles les uns aux autres et une deuxième pluralité d'étirements de tube creux ou fil ou fil élastique (41) qui, dans ladite deuxième pluralité, sont parallèles les uns aux autres mais perpendiculaires à ladite première pluralité d'étirements de tube creux ou fil ou fil élastique (41) ;
ou dans lequel
- ledit diaphragme (8) comprend une première pluralité d'étirements de tube creux ou fil ou fil élastique (41) parallèles les uns aux autres et au moins une deuxième pluralité d'étirements de tube creux ou fil ou fil élastique (41) qui, dans ladite deuxième pluralité, au moins au nombre de une, sont parallèles les uns aux autres mais inclinés par rapport à ladite première pluralité d'étirements de fil élastique (41).

19. Dispositif d'occlusion interseptale pouvant être traversé (1) selon l'une quelconque des revendications 1 à 16, dans lequel
- ladite structure de support (2) est d'un seul tenant ;
- ladite structure de support (2) et ledit diaphragme (8) sont d'un seul tenant ; et/ou dans lequel
- ladite structure de support (2) est en élastomère médical.

20. Dispositif d'occlusion interseptale pouvant être traversé (1) selon l'une quelconque des revendications précédentes, dans lequel
ledit dispositif (1) comprend un diaphragme (8) avec des ponts d'occlusion (21) ayant un corps de pont d'occlusion ondulé (50) ;
ou dans lequel
ledit dispositif (1) comprend un diaphragme (8) avec des ponts d'occlusion (21) ayant un corps de pont d'occlusion ondulé (50), permettant ainsi à chaque pont d'occlusion (21) sollicité par une éventuelle traversée du diaphragme lui-même, par exemple par un dispositif médical, de s'allonger puis de revenir à une position fermée du diaphragme (8) ;
ou dans lequel
ledit dispositif (1) comprend un diaphragme (8) avec des ponts d'occlusion (21) ayant des bords de pont d'occlusion ondulés (22), le bord de pont d'occlusion désignant la portion latérale du pont d'occlusion (21) qui délimite ladite fente allongée (27), permettant ainsi à chaque pont d'occlusion sollicité par une éventuelle traversée du diaphragme par un dispositif médical, de s'allonger, et facilitant, une fois le dispositif retiré, la refermeture des ponts d'occlusion (21) et donc du diaphragme (8) lui-même.

21. Dispositif d'occlusion interseptale pouvant être traversé (1) selon l'une quelconque des revendications 1 à 16 ou 20, dans lequel ledit diaphragme (8) est obtenu par une seule feuille dans laquelle lesdites fentes allongées (27) sont réalisées par des découpes laser.
